(19) Europäisches Patentamt
European Patent Office
Office européen des brevets

(11) **EP 4 160 525 A1**

(12) **EUROPEAN PATENT APPLICATION**
published in accordance with Art. 153(4) EPC

(43) Date of publication:
**05.04.2023 Bulletin 2023/14**

(21) Application number: **21813232.2**

(22) Date of filing: **24.05.2021**

(51) International Patent Classification (IPC):
**G06T 7/00** (2006.01)      **A61B 5/00** (2006.01)
**A61B 5/107** (2006.01)

(52) Cooperative Patent Classification (CPC):
**A61B 5/00; A61B 5/107; G06T 7/00**

(86) International application number:
**PCT/JP2021/019525**

(87) International publication number:
**WO 2021/241475 (02.12.2021 Gazette 2021/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.05.2020   JP 2020092470
17.12.2020   JP 2020209513**

(71) Applicant: **Shiseido Company, Ltd.**
**Chuo-ku**
**Tokyo 104-0061 (JP)**

(72) Inventor: **TAKAI, Eisuke**
**Tokyo 104-0061 (JP)**

(74) Representative: **Ter Meer Steinmeister & Partner**
**Patentanwälte mbB**
**Nymphenburger Straße 4**
**80335 München (DE)**

(54) **SAGGING EVALUATION METHOD, DEVICE, PROGRAM, AND SYSTEM**

(57)    Factors that cause a sagging appearance are found, and sagging is evaluated based on the found factors. A method according to one embodiment of the present disclosure includes a step of acquiring a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position; a step of calculating an intrafacial movement induced sag amount and an anteroposterior buccal sag amount from the gravity-induced prominence amount and the gravity-induced hollowness amount; and a step of evaluating a type of sagging and a degree thereof based on the intrafacial movement induced sag amount and the anteroposterior buccal sag amount as indices.

FIG.16

START OF EVALUATION PROCESSING

ACQUIRE GRAVITY-INDUCED PROMINENCE AMOUNT AND GRAVITY-INDUCED HOLLOWNESS AMOUNT — S11

CALCULATE INTRAFACIAL MOVEMENT INDUCED SAG AMOUNT AND ANTEROPOSTERIOR BUCCAL SAG AMOUNT — S12

OUTPUT RESULT — S13

END OF EVALUATION PROCESSING

EP 4 160 525 A1

**Description**

**TECHNICAL FIELD**

The present disclosure relates to sagging evaluation methods, devices, programs, and systems.

**BACKGROUND ART**

Conventionally, a method for evaluating facial sagging is known. For example, in Patent Document 1, a geometric area that has been defined on a skin surface by a closed curve is measured in two postures, that is, in a horizontal position and a vertical position, and a ratio or a difference between the respective measured areas of the two postures are measured as an amount of sagging skin. In this manner, conventional evaluation of sagging is performed based on an assumption that sagging appearance of the face is caused by the drooping of the facial form in the direction of gravity. A method of evaluating sagging on a six-grade score from 0 to 5 based on sagging found in each area (upper cheek area, lower cheek area, and lateral cheek area) of the face where sagging advances in proportion to age is reported in Non-Patent Document 1.

**RELATED-ART DOCUMENTS**

**PATENT DOCUMENTS**

Patent Document 1: Japanese Patent No. 6473959

**NON-PATENT DOCUMENTS**

Non-Patent Document 1: T. Ezure, J. Hosoi, S. Amano, and T. Tsuchiya, Skin Research and Technology 2009; 15: 299-305

**SUMMARY OF THE INVENTION**

**PROBLEM TO BE SOLVED BY THE INVENTION**

However, conventionally, the reason as to why the face appears to sag has not been sufficiently understood. Hence, an evaluation based on appropriate factors that cause a sagging appearance has been desired.
Therefore, an object of the present disclosure is to find factors that cause a sagging appearance and to perform evaluation based on the found factors.

**MEANS TO SOLVE THE PROBLEM**

A method according to one aspect of the present disclosure includes a step of acquiring a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position; a step of calculating an intrafacial movement induced sag amount and an anteroposterior buccal sag amount from the gravity-induced prominence amount and the gravity-induced hollowness amount; and a step of evaluating a type of sagging and a degree thereof based on the intrafacial movement induced sag amount and the anteroposterior buccal sag amount as indices.

**EFFECTS OF THE INVENTION**

According to the present disclosure, factors that cause a sagging appearance can be found, and an evaluation can be performed based on the found factors.

**BRIEF DESCRIPTION OF THE DRAWINGS**

FIG. 1 is a view for explaining gravity-induced prominence according one embodiment of the present disclosure;
FIG. 2 is a view for explaining gravity-induced hollowness according one embodiment of the present disclosure;

FIG. 3 is a graph illustrating the relationship between gravity-induced prominence and gravity-induced hollowness according to one embodiment of the present disclosure;

FIG. 4 is a view for explaining an intrafacial movement induced sag according to one embodiment of the present disclosure;

FIG. 5 is a view for explaining an anteroposterior buccal sag according to one embodiment of the present disclosure;

FIG. 6 is a table comparing the intrafacial movement induced sag and the anteroposterior buccal sag according to one embodiment of the present disclosure;

FIG. 7 is a graph illustrating the relationship between gravity-induced prominence and a visual score according to one embodiment of the present disclosure;

FIG. 8 is a view for explaining a sagging skin coefficient according to one embodiment of the present disclosure;

FIG. 9 is a view comparing the correlation between the sagging skin coefficient and the visual score, the correlation between the intrafacial movement induced sag and the visual score, and the correlation between anteroposterior buccal sag and the visual score according to one embodiment of the present disclosure;

FIG. 10 is a view comparing the correlation between the sagging skin coefficient and age, the correlation between the intrafacial movement induced sag and age, and the correlation between anteroposterior buccal sag and age according to one embodiment of the present disclosure;

FIG. 11 is a block diagram of an evaluation system according to one embodiment of the present disclosure;

FIG. 12 is a functional block diagram of an evaluation device according to one embodiment of the present disclosure;

FIG. 13 is a functional block diagram of an image capturing terminal according to one embodiment of the present disclosure;

FIG. 14 is a functional block diagram of an analysis terminal according to one embodiment of the present disclosure;

FIG. 15 is a sequence diagram of evaluation processing according to one embodiment of the present disclosure;

FIG. 16 is a flowchart of the evaluation processing according to one embodiment of the present disclosure;

FIG. 17 is a flowchart of the evaluation processing for the intrafacial movement induced sag according to one embodiment of the present disclosure;

FIG. 18 is a flowchart of the evaluation processing for the intrafacial movement induced sag according to one embodiment of the present disclosure;

FIG. 19 is a flowchart of the evaluation processing for the anteroposterior buccal sag according to one embodiment of the present disclosure;

FIG. 20 is a flowchart of the evaluation processing for the anteroposterior buccal sag according to one embodiment of the present disclosure;

FIG. 21 is a flowchart of the evaluation processing for the sagging skin coefficient according to one embodiment of the present disclosure;

FIG. 22 is a flowchart of the evaluation processing for the visual score according to one embodiment of the present disclosure;

FIG. 23 is a block diagram illustrating an example of the hardware configuration of the evaluation device and the analysis terminal according to one embodiment of the present disclosure;

FIG. 24 is a view illustrating a case comparing visual scores acquired from actual visual evaluations and visual scores calculated by equation (4) upon calculating the sagging skin coefficient by using one of the skin viscoelasticity parameters acquired by a viscoelasticity device according to one embodiment of the present disclosure;

FIG. 25 is a view illustrating a case comparing visual scores acquired from actual visual evaluations and visual scores calculated by equation (4) upon calculating the sagging skin coefficient by using two of the skin viscoelasticity parameters acquired by the viscoelasticity device according to one embodiment of the present disclosure;

FIG. 26 is a view illustrating three-dimensional face images acquired in the same period of time, on the same date, and in the same place according to one embodiment of the present disclosure;

FIG. 27 is a graph illustrating the variation in the gravity-induced prominence and the gravity-induced hollowness according to one embodiment of the present disclosure;

FIG. 28 is a view for explaining iterative acquisition of three-dimensional face images according to one embodiment of the present disclosure;

FIG. 29 is a graph for explaining the representative values of at least one of a gravity-induced prominence amount $VC_{swelling}$, a gravity-induced hollowness amount $VC_{shrinking}$, an intrafacial movement induced sag amount $S\varepsilon$, or an anteroposterior buccal sag amount $S_c$ based on values calculated by using the three-dimensional face images that were acquired iteratively;

FIG. 30 is a graph for explaining daily differences between the representative values of at least one of the gravity-induced prominence amount $VC_{swelling}$, the gravity-induced hollowness amount $VC_{shrinking}$, the intrafacial movement induced sag amount $S_f$, or the anteroposterior buccal sag amount $S_c$ based on values calculated by using the three-dimensional face images that were acquired iteratively; and

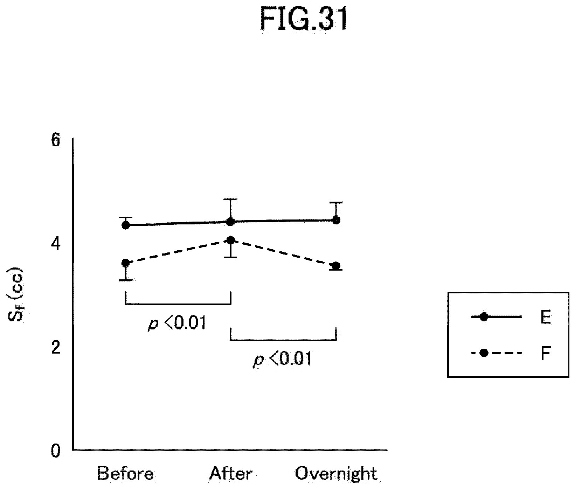

FIG. 31 is a graph for explaining a result of comparing subjects or groups of subjects who have undergone different strengths of treatments or comparing those who have undergone treatment with those who have not for the purpose of measuring the effect of the treatments on sagging by acquiring the representative values of least one of the gravity-induced prominence amount $VC_{swelling}$, the gravity-induced hollowness amount $VC_{shrinking}$, the intrafacial movement induced sag amount $S_f$, or the anteroposterior buccal sag amount $S_c$ based on values calculated by using the three-dimensional face images that were acquired iteratively.

## MODE FOR CARRYING OUT THE INVENTION

Embodiments of the present disclosure will be described hereinafter with reference to the drawings.

<Description of Terms>

- "Three-dimensional face image" refers to an image expressing the three-dimensional shape of a face of a person who is the subject of sag evaluation.
- "Horizontal position" refers to a state where the median plane of the face rests at right angles to the direction of gravity.
- "Vertical position" refers a state where the median plane of the face rests parallel to the direction of gravity.
- "Visual score (to be also referred to as visual evaluation or $S_{ss}$)" is a quantified value representing a visual valuation of sagging. As reported in Non-Patent Document 1, a visual score is calculated based on determination by an evaluator as to which of reference photographs 1 through 5 the face of a person subject to sag evaluation is closest. For example, the visual score is calculated from an oblique side face image (an image obtained by capturing the face at an angle of 45° to the left and right from the front of the face) when the face of the person subject to the sag evaluation is in a vertical position (that is, in a state where the median plane of the face rests parallel to the direction of gravity). Note that the visual score of the face of the person subject to sag evaluation may be calculated by a trained model generated from machine learning by using the oblique side face images for which visual scores have been determined as the training data. The visual score is determined for each of the upper cheek area, the lower cheek area, and the lateral cheek area, but the visual score may also be newly generated by averaging the scores for one of these areas. $S_{ss}$ is acquired by averaging, in particular, the visual scores of the upper cheek area and the lower cheek area, and indicates a sagging appearance in the upper and lower cheek areas.

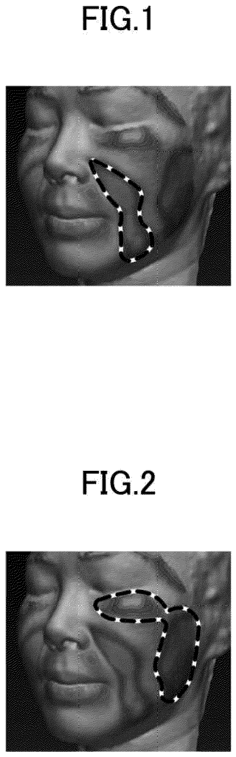

Factors that affect a sagging appearance (that is, the visual score) will be described hereinafter with reference to FIGs. 1 to 10.

First, the areas of the face where the volume changes when the face of the person is in the horizontal position (that is, in a state where the median plane of the face is stationary at right angles to the direction of gravity) and when the face of the person is in the vertical position (that is, a state where the median plane of the face rests parallel to the direction of gravity) will be described with reference to FIGs. 1 and 2. As will be described hereinafter, the changes in volume were calculated by using images (to be also referred to as three-dimensional face images) each expressing the three-dimensional shape of the face of the person subject to sag evaluation.

<Gravity-induced Prominence>

FIG. 1 is a view for explaining gravity-induced prominence according to one embodiment of the present disclosure. A three-dimensional face image captured when the subject's face is in the horizontal position and a three-dimensional face image captured when the subject's face is in the vertical position were acquired. When the three-dimensional shape in the horizontal position was compared with the three-dimensional shape captured in the vertical position, the volume of the area encircled by a broken line in FIG. 1 was larger in the three-dimensional shape in the vertical position. Assume that a gravity-induced prominence amount $VC_{swelling}$ is the difference between the volumes in the two positions (that is, the horizontal position and the vertical position) when the volume of the area in the three-dimensional shape is larger in the vertical position in this manner.

<Gravity-induced Hollowness>

FIG. 2 is a view for explaining gravity-induced hollowness according to one embodiment of the present disclosure. A three-dimensional face image captured when the subject's face is in the horizontal position and a three-dimensional face image captured when the subject's face is in the vertical position were acquired. When the three-dimensional shape in the horizontal position was compared with the three-dimensional shape in the vertical position, the volume of the area encircled by a broken line in FIG. 2 was smaller in the three-dimensional shape in the vertical position. Assume that a gravity-induced hollowness amount $VC_{shrinking}$ is the difference between the volumes in the two positions (that is, the horizontal position and the vertical position) when the volume of the area in the three-dimensional shape is smaller in the vertical position in this manner.

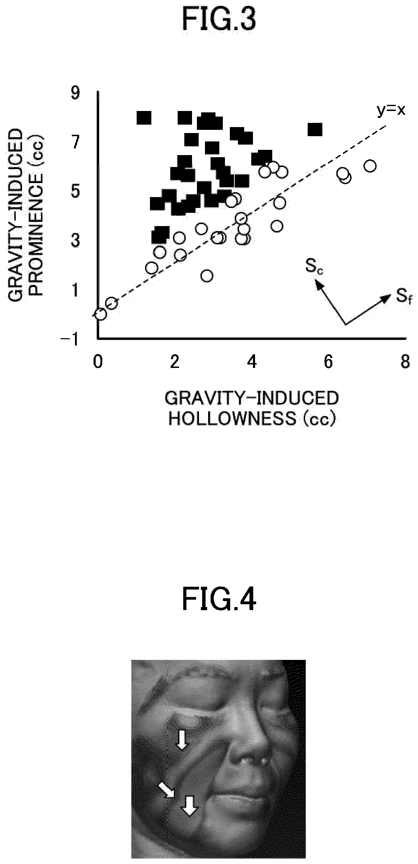

FIG. 3 is a graph illustrating the relationship between two types of sagging ("intrafacial movement induced sag amount $S_f$" and "anteroposterior buccal sag amount $S_c$", which are to be described in detail later) that occur on the face and a phenomenon in which the shape of the face changes due to the influence of gravity from the gravity-induced prominence and the gravity-induced hollowness according to one embodiment of the present disclosure. The two types of sagging (that is, the "intrafacial movement induced sag amount $S_f$" and the "anteroposterior buccal sag amount $S_c$") can be calculated from the gravity-induced prominence amount $VC_{swelling}$ of FIG. 1 and the gravity-induced hollowness amount $VC_{shrinking}$ of FIG. 2 by equation (1) below. Letting the gravity-induced hollowness amount $VC_{shrinking}$ be the x-axis and the gravity-induced prominence amount $VC_{swelling}$ be the y-axis, the two types of sagging (the intrafacial movement induced sag amount $S_f$ and the anteroposterior buccal sag amount $S_c$ can be quantified by converting the coordinates counterclockwise by 45° as follows:

$$\begin{pmatrix} S_f \\ S_c \end{pmatrix} = \begin{pmatrix} \cos\theta & \sin\theta \\ -\sin\theta & \cos\theta \end{pmatrix} \begin{pmatrix} VC_{shrinking} \\ VC_{swelling} \end{pmatrix} \quad \cdots (1)$$

where $VC_{shrinking}$ is the gravity-induced hollowness amount, $VC_{swelling}$ is the gravity-induced prominence amount, $S_f$ is the intrafacial movement induced sag amount, $S_c$ is the anteroposterior buccal sag amount, and $\theta$ is $\pi/4$.

The intrafacial movement induced sag and the anteroposterior buccal sag will be described in detail hereinafter with reference to FIGs. 4 to 6.

FIG. 4 is a view for explaining the intrafacial movement induced sag according to one embodiment of the present disclosure. As illustrated in FIG. 4, the intrafacial movement induced sag is a sag from the upper cheek area to the lower cheek area.

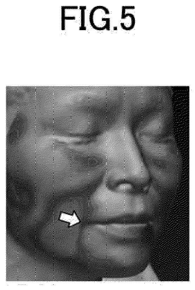

FIG. 5 is a view for explaining the anteroposterior buccal sag according to one embodiment of the present disclosure. As illustrated in FIG. 5, the anteroposterior buccal sag is a sag other than the sag from the upper cheek area to the lower cheek area (for example, a sag around the mouth in which the cheek moves anteroposteriorly from the side of the mouth due to deformation of the cheek).

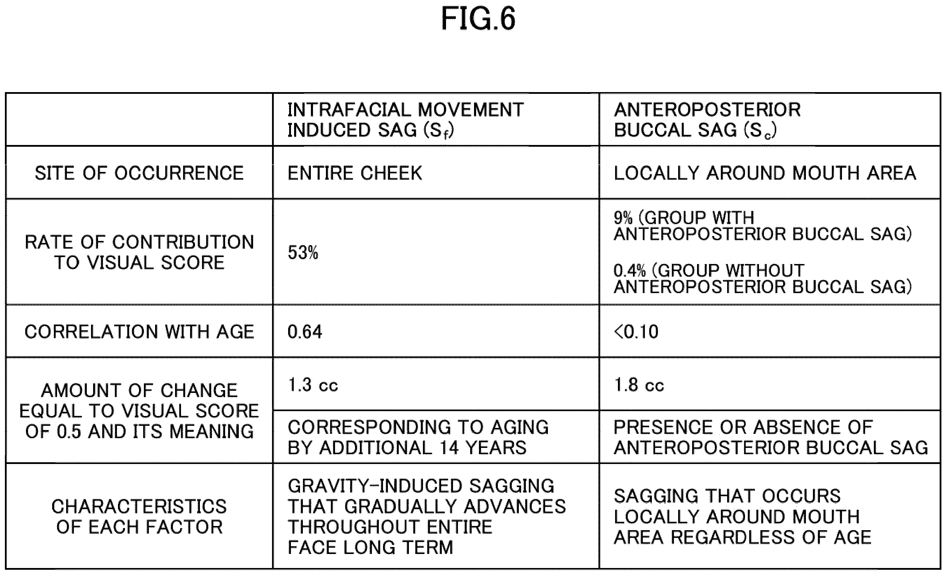

FIG. 6 is a table comparing the intrafacial movement induced sag and the anteroposterior buccal sag according to one embodiment of the present disclosure. Each compared item will be described hereinafter.

<Site of Occurrence>

The intrafacial movement induced sag occurs on the entire area of the cheek. In contrast, the anteroposterior buccal sag occurs locally (around the mouth).

<Rate of Contribution to Visual Score>

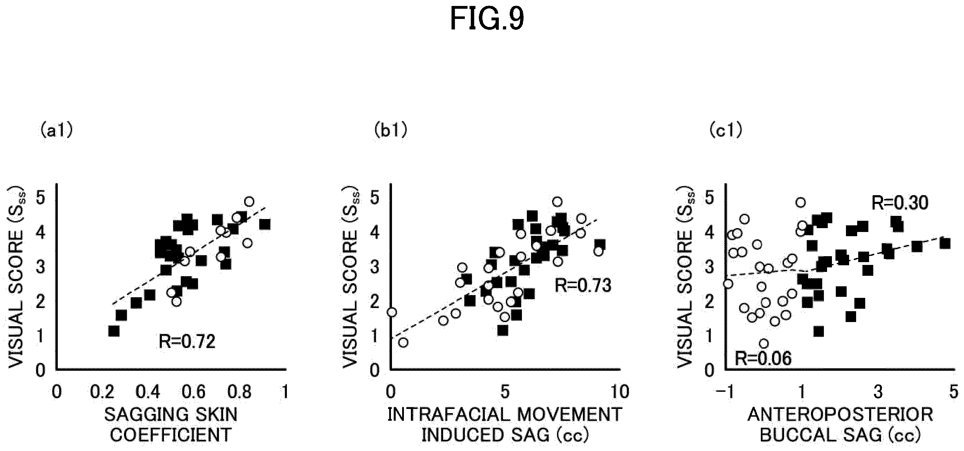

Based on a correlation R between the visual score and the intrafacial movement induced sag to be described with reference to FIG. 9, a rate of contribution R2 (also referred to as a coefficient of determination) of the intrafacial movement induced sag with respect to the visual score was 53%. In contrast, based on the correlation R between the visual score and the anteroposterior buccal sag to be described with reference to FIG. 9, the rate of contribution R2 (also referred to as the coefficient of determination) of the anteroposterior buccal sag with respect to the visual score was 9% for a group with the anteroposterior buccal sag and 0.4% for a group without the anteroposterior buccal sag. Note that the visual score is a score obtained by quantifying the visual evaluation of sagging.

<Correlation with Age>

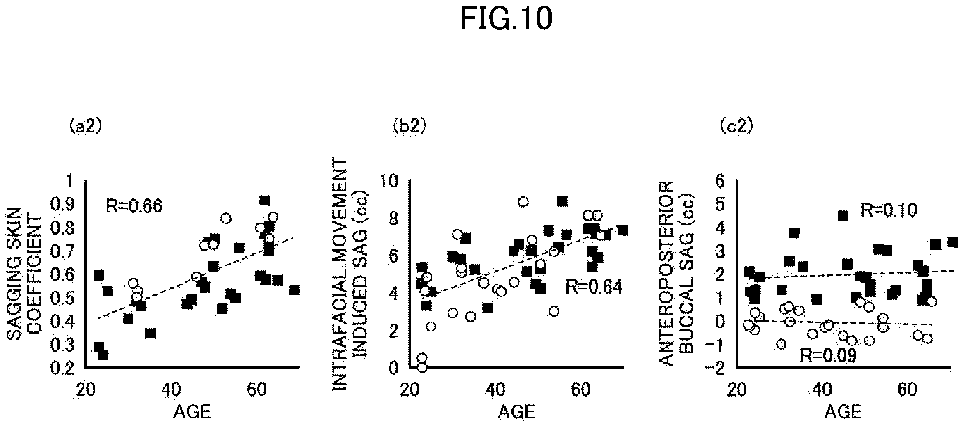

Based on the relationship between age and the intrafacial movement induced sag to be described with reference to FIG. 10, there was a correlation (correlation coefficient of 0.64) between age and the intrafacial movement induced sag. In contrast, based on the relationship between age and the anteroposterior buccal sag to be described with reference to FIG. 10, there was, surprisingly, no correlation between age and the anteroposterior buccal sag. Thus, it can be said that the anteroposterior buccal sag is caused by individual differences regardless of age.

<Change in Volume per Visual Evaluation of 0.5 and Its Meaning>

Based on the relationship between age, the visual score, and the intrafacial movement induced sag to be described with reference to FIGs. 9 and 10, the change in the volume of intrafacial movement induced sag per visual evaluation (visual score) of 0.5 was 1.3 cc. This volume corresponds to aging by an additional 14 years of age. In contrast, based on the relationship between age, the visual score, and the anteroposterior buccal sag to be described with reference to FIGs. 9 and 10, the change in the volume of anteroposterior buccal sag per visual evaluation (visual score) of 0.5 was 1.8 cc. This volume represents the presence or absence of the anteroposterior buccal sag. Note that the visual score is a score obtained by quantifying the visual evaluation of sagging.

<Quality of Sagging>

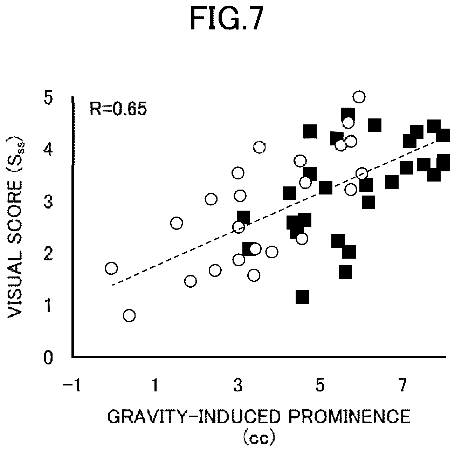

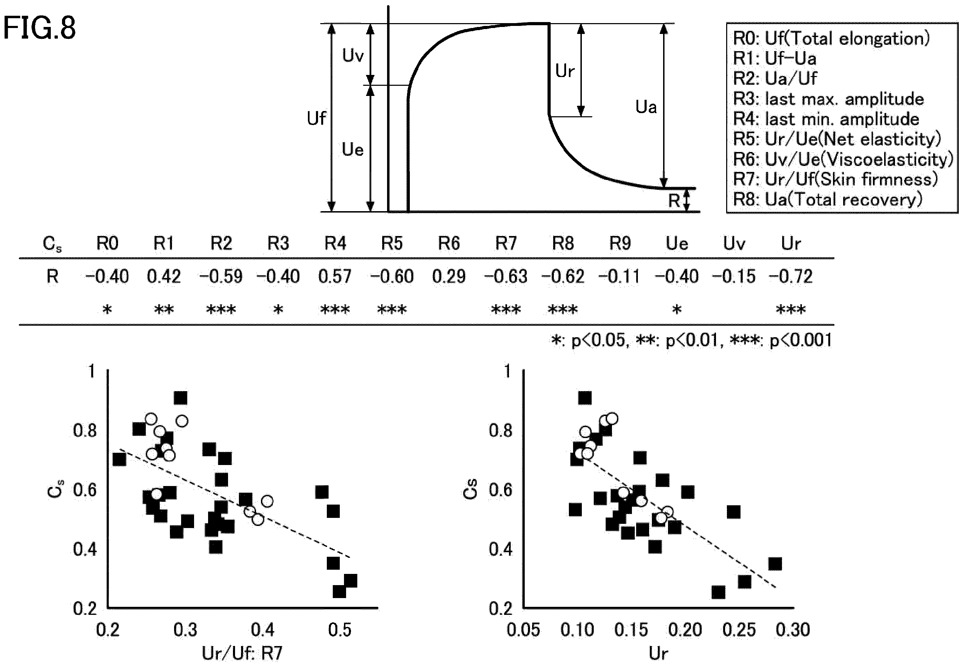

The intrafacial movement induced sag is sagging that gradually advances over a long period on the entire face due to gravity. In contrast, the anteroposterior buccal sag is sagging that occurs locally (around the mouth area) regardless of age due to gravity.
Two factors (that is, the two types of sagging referred to as an "intrafacial movement induced sag" and an "anteroposterior buccal sag") that cause a sagging appearance were found in this manner. Further, the two factors (that is, the two types of sagging referred to as the "intrafacial movement induced sag" and the "anteroposterior buccal sag") that cause a sagging appearance can be evaluated in accordance with the change in the volume of the face of a person between when the face of the person is in the horizontal position and when the face of the person is in the vertical position.
Furthermore, a sagging skin coefficient, which is a third factor that differs from the "intrafacial movement induced sag" and the "anteroposterior buccal sag" but also contributes to a sagging appearance, will be described with reference to FIGs. 7 and 8.
FIG. 7 is a graph illustrating the relationship between the visual evaluation (visual score) and the gravity-induced prominence according to one embodiment of the present disclosure. Some subjects have different visual scores $S_{ss}$ even when the gravity-induced prominence amount $VC_{swelling}$ is the same. For example, looking at subjects whose gravity-induced prominence amount is around 5 cc, their visual scores vary from about 1 to about 4. The sagging skin coefficient $C_s$ can be defined based on the relationship between the gravity-induced prominence amount $VC_{swelling}$ and the visual score $S_{ss}$ by equation (2) as follows. Since $VC_{swelling} \neq 0$ is required to directly obtain $C_s$ from the following equation (2), for each $C_s$ indicated in FIG. 8, the sagging skin coefficient was calculated for each subject whose $VC_{swelling}$ was less than 3.5 cc, which is the gravity-induced prominence amount that a 30-year-old can have on average. As will be described later, in addition to directly calculating the sagging skin coefficient from the gravity-induced prominence amount $VC_{swelling}$ and the visual score $S_{ss}$ by the following equation (2), the sagging skin coefficient may also be calculated from skin viscoelasticity parameters by using equations (5) and (6) based on the property that the sagging skin coefficient $C_s$ is well correlated with the skin viscoelasticity parameters.

$$S_{ss} = C_s \times VC_{swelling} \quad \cdots (2)$$

Also, from the relationship between the gravity-induced prominence amount $VC_{swelling}$, the intrafacial movement induced sag amount $S_f$, and the anteroposterior buccal sag amount $S_c$, equation (3) was found as follows.

$$VC_{swelling} = \frac{1}{\sqrt{2}}(S_c + S_f) \quad \cdots (3)$$

Based on equations (2) and (3) described above, the relationship between a sagging appearance (that is, the visual score $S_{ss}$), the sagging skin coefficient $C_s$, the intrafacial movement induced sag amount $S_f$, and the anteroposterior buccal sag amount $S_c$ can be expressed by equation (4) as follows.

$$S_{ss} = C_s \times \frac{1}{\sqrt{2}}(S_c + S_f) \quad \cdots (4)$$

FIG. 8 is a view for explaining the sagging skin coefficient according to one embodiment of the present disclosure. When the correlation between the sagging skin coefficient $C_s$ and the parameters of a viscoelasticity acquisition device was examined, the sagging skin coefficient $C_s$ was correlated with the skin viscoelasticity parameters acquired by the viscoelasticity acquisition device. That is, it was found that a person with a high sagging skin coefficient $C_s$ is a person without skin firmness, and sagging appears emphasized on such skin even if it is the same sagging that is caused by gravity.

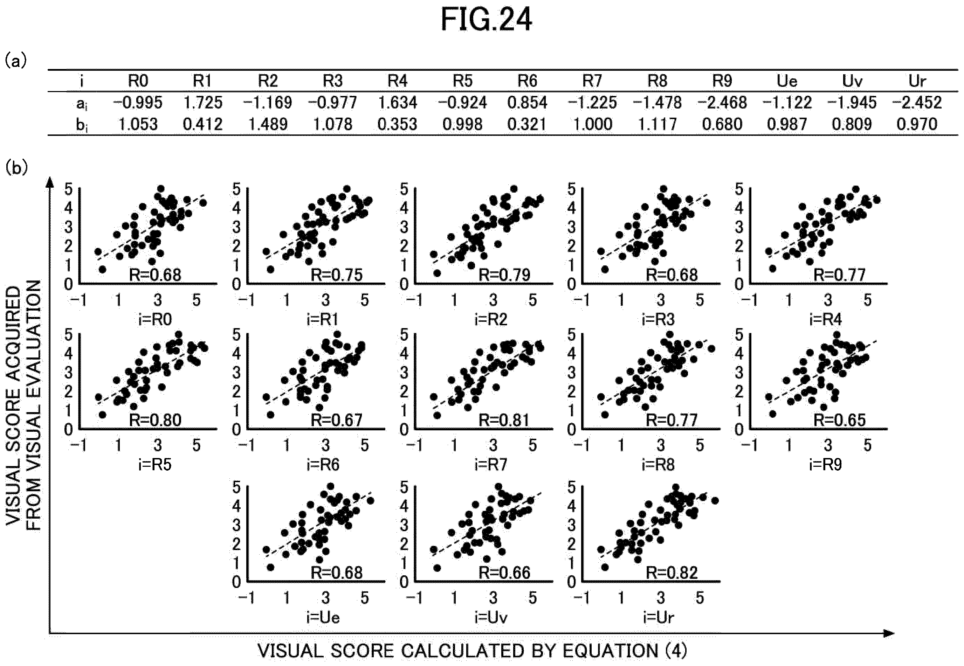

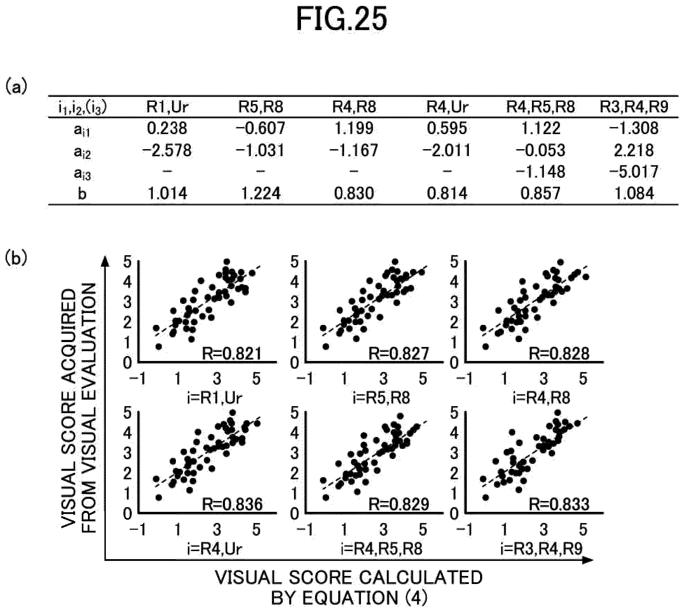

Note that FIG. 24 illustrates a case where the sagging skin coefficient is calculated from one of the skin viscoelasticity parameters acquired by the viscoelasticity acquisition device according to one embodiment of the present disclosure, and the visual score calculated by using equation (4) is compared with a visual score acquired from an actual visual evaluation. FIG. 25 illustrates a case where the sagging skin coefficient is calculated from two or more of skin viscoelasticity parameters acquired by the viscoelasticity acquisition device according to one embodiment of the present disclosure, and the visual score calculated by using equation (4) is compared with a visual score acquired from an actual visual evaluation. Comparing FIG. 24 with FIG. 25, it can be seen that the correlation coefficient is higher in FIG. 25 (in which the sagging skin coefficient is calculated by using two or more skin viscoelasticity parameters) than in FIG. 24 (in which the sagging skin coefficient is calculated by using one of the skin viscoelasticity parameters).

FIG. 24(a) indicates the fitting coefficients calculated from the skin viscoelasticity parameter by using equation (5), as illustrated below, based on the property that the sagging skin coefficient $C_s$ is well correlated with skin viscoelasticity parameters. $a_i$ and $b_i$ were acquired from $E_i$ and $C_s$ of a group of subjects with a $VC_{swelling} > 3.5$ cc, which is the gravity-induced prominence amount that a 30-year-old can have on average.

$$C_s = a_i E_i + b_i \quad (\text{i=R0} \sim \text{R9,Ue,Uv,Ur}) \cdots (5)$$

where E is a skin viscoelasticity parameter, and a and b are fitting coefficients.

In FIG. 24(b), the sagging skin coefficient $C_s$ is acquired from the skin viscoelasticity parameter $E_i$ of each subject by using $a_i$ and $b_i$ indicated in FIG. 24(a), and the visual score $S_{ss}$ that is calculated by using equation (4) compared with the actual visual score $S_{ss}$ that is acquired by using reference photographs as in Non-Patent Document 1. Although the correlation coefficient between the visual score $S_{ss}$ calculated by using equation (4) and the actual visual score $S_{ss}$ varies depending on the skin viscoelasticity parameter that is used, it can be seen that using an appropriate parameter allows the visual score $S_{ss}$ to be accurately calculated from three factors, which are the "sagging skin coefficient $C_s$", the "intrafacial movement induced sag amount $S_f$", and the "anteroposterior buccal sag amount $S_c$".

FIG. 25(a) indicates the fitting coefficients calculated from two or more skin viscoelasticity parameters by using equation (6), as illustrated below, based on the property that the sagging skin coefficient $C_s$ is well correlated with skin viscoelasticity parameters. $a_i$ and b were acquired from $E_i$ and $C_s$ of a group of subjects with $VC_{swelling} > 3.5$ cc, which is the gravity-induced prominence amount that a 30-year-old can have on average.

$$C_s = \sum_i (a_i E_i) + b \quad (\text{i=R0} \sim \text{R9,Ue,Uv,Ur}) \cdots (6)$$

where E is a skin viscoelasticity parameter, and a and b are fitting coefficients.

In FIG. 25(b), the sagging skin coefficient $C_s$ is acquired from the skin viscoelasticity parameter $E_i$ of each subject by using $a_i$ and b indicated in FIG. 25(a), and the visual score $S_{ss}$ that is calculated by using equation (4) is compared with the actual visual score $S_{ss}$ that is acquired by using reference photographs as in Non-Patent Document 1. The correlation coefficient between the visual score $S_{ss}$ calculated by using equation (4) and the actual visual score $S_{ss}$ varies depending on the skin viscoelasticity parameters that are used. However, it can be seen that using appropriate parameters allows the visual score $S_{ss}$ to be calculated from the three factors, which are the "sagging skin coefficient $C_s$", the "intrafacial movement induced sag amount $S_f$", and the "anteroposterior buccal sag amount $S_c$", more accurately in a case where two or more skin viscoelasticity parameters are used than in a case where one skin viscoelasticity parameter is used.

In this manner, a sagging appearance (that is, the visual score $S_{ss}$) is composed of three factors, that is, the "sagging skin coefficient $C_s$", the "intrafacial movement induced sag amount $S_f$", and the "anteroposterior buccal sag amount $S_c$".

The "sagging skin coefficient $C_s$", the "intrafacial movement induced sag amount $S_f$", and the "anteroposterior buccal sag amount $S_c$" will be compared and described below with reference to FIGs. 9 and 10.

FIG. 9 is a view comparing the correlation between the sagging skin coefficient $C_s$ and the visual evaluation

(visual score $S_{ss}$), the correlation between the intrafacial movement induced sag amount $S_f$ and the visual score $S_{ss}$, and the correlation between the anteroposterior buccal sag amount $S_c$ and the visual score $S_{ss}$ according to one embodiment of the present disclosure.

As indicated in a graph a1 illustrated on the left side of FIG. 9, the correlation coefficient between the sagging skin coefficient $C_s$ and the visual score $S_{ss}$ is 0.72, and the coefficient of determination is 0.5184. Hence, it can be said the rate of contribution of the sagging skin coefficient $C_s$ with respect to the visual score $S_{ss}$ is 52%.

As indicated in a graph b1 illustrated in the middle of FIG. 9, the correlation coefficient between the intrafacial movement induced sag amount $S_f$ and the visual score $S_{ss}$ is 0.73, and the coefficient of determination is 0.5305. Hence, it can be said the rate of contribution of the intrafacial movement induced sag amount $S_f$ with respect to the visual score $S_{ss}$ is 53%.

As indicated in a graph c1 illustrated on the right side of FIG. 9, the correlation coefficient between the anteroposterior buccal sag amount $S_c$ and the visual score $S_{ss}$ is 0.30 for the group with the anteroposterior buccal sag, and the coefficient of determination is 0.09. Hence, it can be said that the rate of contribution of the anteroposterior buccal sag amount with respect to the visual score $S_{ss}$ is 9%.

Note that the sum of the rate of contribution of the sagging skin coefficient $C_s$, the rate of contribution of the intrafacial movement induced sag amount $S_f$, and the rate of contribution of the anteroposterior buccal sag amount $S_c$ is 114%. This is due to multicollinearity (a strong correlation between explanatory variables) between the sagging skin coefficient $C_s$ and the intrafacial movement induced sag amount $S_f$ that arises from the strong correlation that both the sagging skin coefficient $C_s$ and the intrafacial movement induced sag amount $S_f$ have with age as illustrated in FIG. 10.

FIG. 10 is a view comparing the correlation between the sagging skin coefficient $C_s$ and age, the correlation between the intrafacial movement induced sag amount $S_f$ and age, and the correlation between the anteroposterior buccal sag amount $S_c$ and age according to one embodiment of the present disclosure.

As indicated in a graph a2 illustrated on the left side of FIG. 10, the sagging skin coefficient $C_s$ increases as the age increases, and the correlation coefficient is 0.66. Hence, it can be said that the sagging skin coefficient $C_s$, as a factor constituting a sagging appearance, is a phenomenon caused by aging.

As indicated in a graph b2 illustrated in the middle of FIG. 10, the intrafacial movement induced sag amount $S_f$ increases as the age increases, and the correlation coefficient is 0.64. Hence, it can be said that the intrafacial movement induced sag amount $S_\varepsilon$, as a factor constituting a sagging appearance, is a phenomenon caused by aging.

As indicated in a graph c2 illustrated on the right side of FIG. 10, subjects with a large anteroposterior buccal sag amount $S_c$ (group with the anteroposterior buccal sag) and subjects with a small anteroposterior buccal sag amount $S_c$ (group without anteroposterior buccal sag) are distributed among all ages. Hence, it can be said that the anteroposterior buccal sag amount $S_c$, as a factor constituting a sagging appearance, is a phenomenon caused by aging.

<Overall System Configuration>

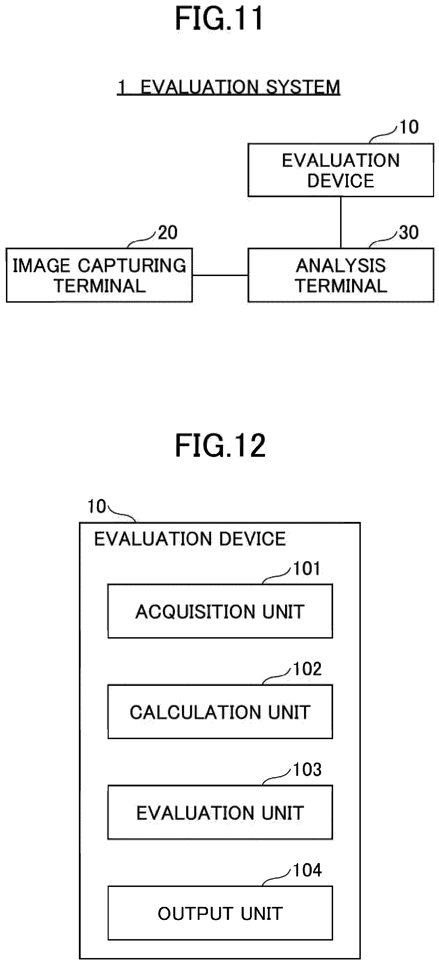

FIG. 11 is a block diagram of an evaluation system 1 according to one embodiment of the present disclosure. The evaluation system 1 (to be also called an evaluation system for evaluating the type of sagging and the degree thereof) according to one embodiment of the present disclosure includes an evaluation device 10 (to be also called an evaluation device for evaluating the type of sagging and the degree thereof), an image capturing terminal 20, and an analysis terminal 30. The evaluation device 10 and the analysis terminal 30 can exchange data via any network. Each device will be described hereinafter.

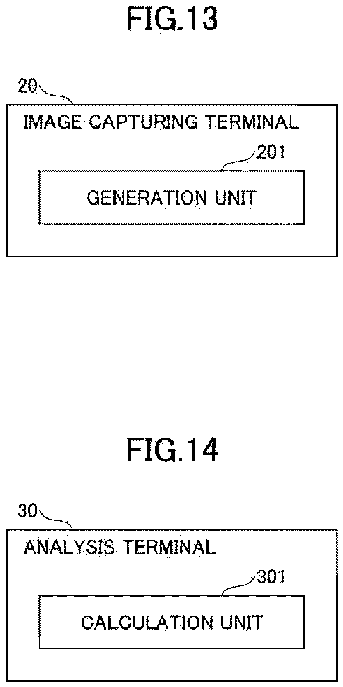

The image capturing terminal 20 is a terminal configured to generate data of an image (three-dimensional face image) expressing the three-dimensional shape of the face of a person subject to sagging evaluation. For example, the image capturing terminal 20 is a measurement device configured to capture a 3D (three-dimensional) image. More specifically, the image capturing terminal 20 generates the three-dimensional face image data of when the face of the person subject to sagging evaluation is in the horizontal position (specifically, when the median plane of the face rests at right angles to the direction of gravity) and the three-dimensional face image data of when the face of the person subject to sagging evaluation is in the vertical position (specifically, when the median plane of the face rests parallel to the direction of gravity). The image capturing terminal 20 will be described in detail later with reference to FIG. 13.

The analysis terminal 30 is a computer (for example, a personal computer, a tablet, a smartphone, or the like) configured to calculate the information used for sagging evaluation based on the data generated by the image capturing terminal 20. More specifically, the analysis terminal 30 calculates the gravity-induced prominence amount $VC_{swelling}$ and the gravity-induced hollowness amount $VC_{shrinking}$ based on the change between the three-dimensional shape of the three-dimensional face image acquired when the face of the person subject to the sagging evaluation is in the horizontal position and the three-dimensional shape of the three-dimensional face image acquired when the face of the person subject to the sagging evaluation is in the vertical position. The analysis terminal 30 will be described in detail later with reference to FIG. 14.

The evaluation device 10 is a computer (for example, a server) configured to evaluate sagging. The evaluation device 10 calculates, for example, the intrafacial movement induced sag amount $S_f$ and the anteroposterior buccal sag amount $S_c$ based on the gravity-induced prominence amount $VC_{swelling}$ and the gravity-induced hollowness amount $VC_{shrinking}$. The evaluation device 10 will be described in detail later with reference to FIG. 12.

Although the evaluation device 10 and the analysis terminal 30 are described as separate computers in FIG. 11, the evaluation device 10 and the analysis terminal 30 may be implemented by a single computer.

FIG. 12 is a functional block diagram of the evaluation device 10 according to one embodiment of the present disclosure. As illustrated in FIG. 12, the evaluation device 10 can include an acquisition unit 101, a calculation unit 102, an evaluation unit 103, and an output unit 104. The evaluation device 10 can also executed a program to function as the acquisition unit 101, the calculation unit 102, the evaluation unit 103, and the output unit 104. Each unit will be described hereinafter.

The acquisition unit 101 acquires the gravity-induced prominence amount $VC_{swelling}$ and the gravity-induced hollowness amount $VC_{shrinking}$ calculated by the analysis terminal 30. The acquisition unit 101 also stores the acquired gravity-induced prominence amount $VC_{swelling}$ and the acquired gravity-induced hollowness amount $VC_{shrinking}$ in a memory so as to enable them to be referred to by the calculation unit 102.

The calculation unit 102 calculates the intrafacial movement induced sag amount $S_f$ and the anteroposterior buccal sag amount $S_c$ from the gravity-induced prominence amount $VC_{swelling}$ and the gravity-induced hollowness amount $VC_{shrinking}$. The calculation unit 102 stores the calculated intrafacial movement induced sag amount $S_f$ and the calculated anteroposterior buccal sag amount $S_c$ in a memory to enable them to be referred to by the evaluation unit 103 and the output unit 104. A method of calculating the intrafacial movement induced sag amount $S_f$ and the anteroposterior buccal sag amount $S_c$ will be described in detail below.

<<Calculation of Intrafacial Movement Induced Sag Amount $S_f$ and Anteroposterior Buccal Sag Amount $S_c$>>

The calculation unit 102 calculates the intrafacial movement induced sag amount $S_f$ and the anteroposterior buccal sag amount $S_c$ by letting the gravity-induced hollowness amount $VC_{shrinking}$ be the x-axis and the gravity-induced prominence amount $VC_{swelling}$ be the y-axis and converting the coordinates by 45° as illustrated in equation (1) as follows.

$$\begin{pmatrix} S_f \\ S_c \end{pmatrix} = \begin{pmatrix} \cos\theta & \sin\theta \\ -\sin\theta & \cos\theta \end{pmatrix} \begin{pmatrix} VC_{shrinking} \\ VC_{swelling} \end{pmatrix} \quad \cdots (1)$$

where $\theta = \pi/4$.

The evaluation unit 103 evaluates the type of sagging and the degree thereof by using the intrafacial movement induced sag amount $S_f$ and the anteroposterior buccal sag amount $S_c$ as indices.

The output unit 104 outputs the value of the intrafacial movement induced sag amount $S_\varepsilon$, the value of the anteroposterior buccal sag amount $S_c$, the type of sagging, and the degree thereof. For example, the output unit 104 can be configured to transmit the value of the intrafacial movement induced sag amount $S_f$, the value of the anteroposterior buccal sag amount $S_c$, the type of sagging, and the degree thereof to the analysis terminal 30 or be configured to display the value of the intrafacial movement induced sag amount $S_f$, the value of the anteroposterior buccal sag amount $S_c$, the type of sagging, and the degree thereof on a display unit of the evaluation device 10.

FIG. 13 is a functional block diagram of the image capturing terminal 20 according to one embodiment of the present disclosure. As illustrated in FIG. 13, the image capturing terminal 20 can include a generation unit 201. The image capturing terminal 20 can also function as the generation unit 201 by executing a program. Details will be described below.

The generation unit 201 generates the three-dimensional face image data when the face of the person subject to the sagging evaluation is in the horizontal position and the three-dimensional face image data when the face of the person subject to the sagging evaluation is in the vertical position. The generation unit 201 transmits the generated data to the analysis terminal 30.

FIG. 14 is a functional block diagram of the analysis terminal 30 according to one embodiment of the present disclosure. As illustrated in FIG. 14, the analysis terminal 30 can include a calculation unit 301. The analysis terminal 30 can also function as the calculation unit 301 by executing a program. Details will be described below.

The calculation unit 301 acquires the data generated by the image capturing terminal 20. Further, the calculation unit 301 calculates the gravity-induced prominence amount $VC_{swelling}$ and the gravity-induced hollowness amount $VC_{shrinking}$ based on the change between the three-dimensional shape of the three-dimensional face image of when the face of the person subject to the sagging evaluation is in the horizontal position and the three-dimensional shape of the three-dimensional face image of when the face of the person subject to the sagging evaluation is in the vertical position that were generated by the image capturing terminal 20. Further, the calculation unit 301 can transmit the calculated gravity-induced prominence amount $VC_{swelling}$ and the calculated gravity-induced hollowness amount $VC_{shrinking}$ to the evaluation device 10. The method of calculating the gravity-induced prominence amount $VC_{swelling}$ and the method of calculating the gravity-induced hollowness amount $VC_{shrinking}$ will be described in detail below.

<<Calculation of Gravity-induced prominence Amount $VC_{swelling}$>>

The calculation unit 301 compares the three-dimensional shape in the horizontal position with the three-dimensional shape in the vertical position. The calculation unit 301 determines that, with respect to the area (the area described in FIG. 1) where the volume is large in the vertical position, the difference between the volumes in both positions (that is, the volume of the area in the horizontal position and the volume of the area in the vertical position) is the gravity-induced prominence amount $VC_{swelling}$.

<<Calculation of Gravity-induced Hollowness Amount $VC_{shrinking}$>>

The calculation unit 301 compares the three-dimensional shape in the horizontal position with the three-dimensional shape in the vertical position. The calculation unit 301 determines that, with respect to the area (the area described in FIG. 2) where the volume is small in the vertical position, the difference between the volumes in both positions (that is, the volume of the area in the horizontal position and the volume of the area in the vertical position) is the gravity-induced hollowness amount $VC_{shrinking}$.

<Method>

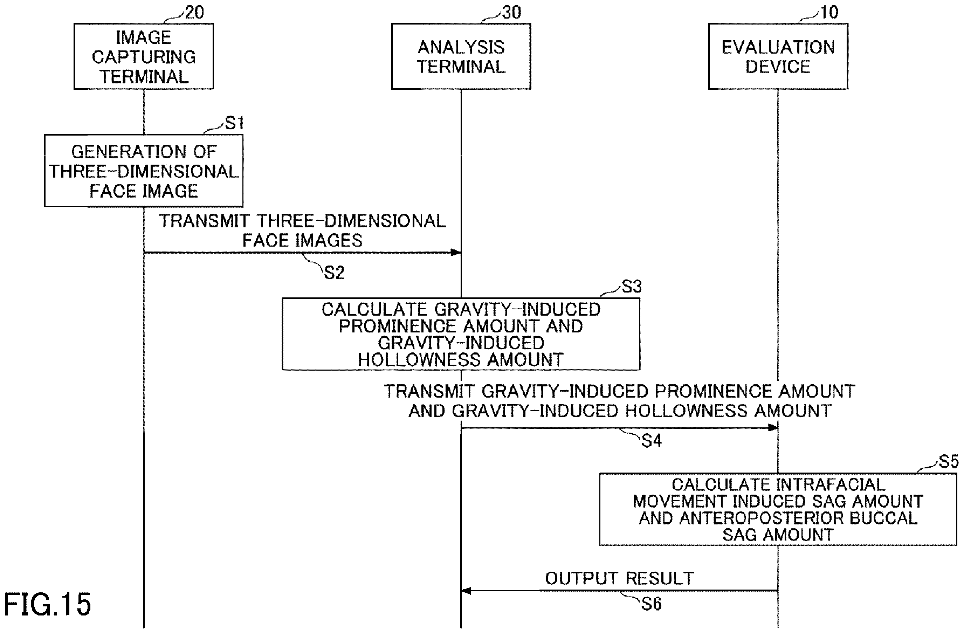

An evaluation method for various types of sagging will be described hereinafter with reference to FIGs. 15 to 22. Note that a part of the method illustrated in FIGs. 15 to 22 may be performed by a person.

FIG. 15 is a sequence diagram of evaluation processing according to one embodiment of the present disclosure. The intrafacial movement induced sag amount $S_f$ and the anteroposterior buccal sag amount $S_c$ are calculated in FIG. 15.

In step 1 (S1), the image capturing terminal 20 generates the three-dimensional face image data of when the face of the person subject to sagging evaluation is in the horizontal position (that is, when the median plane of the face rests at right angles to the direction of gravity) and the three-dimensional face image data of when the face of the person subject to sagging evaluation is in the vertical position (that is, when the median plane of the face rests parallel to the direction of gravity).

In step 2 (S2), the image capturing terminal 20 transmits the data generated in S1 to the analysis terminal 30.

In step 3 (S3), the analysis terminal 30 calculates the gravity-induced prominence amount $VC_{swelling}$ and the gravity-induced hollowness amount $VC_{shrinking}$ based on the change between the three-dimensional shape of the three-dimensional face image in the horizontal position and the three-dimensional shape of the three-dimensional face image in the vertical position acquired in S2.

<<Calculation of Gravity-induced prominence Amount $VC_{swelling}$>>

More specifically, the analysis terminal 30 compares the three-dimensional shape in the horizontal position with the three-dimensional shape in the vertical position. The analysis terminal 30 determines that, with respect to an area (the area described in FIG. 1) where the volume is large in the vertical position, the difference between the volumes in both positions (that is, the volume of the area in the horizontal position and the volume of the area in the vertical position) is the gravity-induced prominence amount $VC_{swelling}$.

<<Calculation of Gravity-induced Hollowness Amount $VC_{shrinking}$>>

More specifically, the analysis terminal 30 compares the three-dimensional shape in the horizontal position with the three-dimensional shape in the vertical position. The analysis terminal 30 determines that, with respect to the area (the area described in FIG. 2) where the volume is small in the vertical position, the difference between the volumes in both positions (that is, the volume of the area in the horizontal position and the volume of the area in the vertical position) is the gravity-induced hollowness amount $VC_{shrinking}$.

In step 4 (S4), the analysis terminal 30 transmits the gravity-induced prominence amount $VC_{swelling}$ and the gravity-induced hollowness amount $VC_{shrinking}$ calculated in S3 to the evaluation device 10.

In step 5 (S5), the evaluation device 10 calculates the intrafacial movement induced sag amount $S_f$ and the anteroposterior buccal sag amount $S_c$ from the gravity-induced prominence amount $VC_{swelling}$ and the gravity-induced hollowness amount $VC_{shrinking}$ acquired in S4. More specifically, the evaluation device 10 calculates the intrafacial movement induced sag amount $S_f$ and the anteroposterior buccal sag amount $S_c$ from the gravity-induced prominence amount $VC_{swelling}$ and the gravity-induced hollowness amount $VC_{shrinking}$ based on the coordinate conversion by equation (1) described above.

Note that the evaluation device 10 can be configured to evaluate the type of sagging and the degree thereof by using the calculated intrafacial movement induced sag amount $S_f$ and the calculated anteroposterior buccal sag amount $S_c$ as indices. For example, the evaluation device 10 can evaluate the type of sagging as follows by setting the proportion of intrafacial movement induced sag amount $S_{\varepsilon}$ as the proportion of sagging caused by aging and setting the proportion of anteroposterior buccal sag amount $S_c$ as the proportion of sagging caused by individual differences.

> TYPE (intrafacial movement induced sag, anteroposterior buccal sag)
> TYPE 1 (present, absent): aging-induced sagging type
> TYPE 2 (absent, present): partial sagging type
> TYPE 3 (present, present): sagging face type
> TYPE 4 (absent, absent): zero presence of sagging type

The intrafacial movement induced sag may also be compared with the average for the age of the subject and the result may be indicated by a degree such as high, medium, and low as illustrated below.

> TYPE 1 (medium): an age-appropriate sagging type
> TYPE 2 (low): a youthful appearance sagging type
> TYPE 3 (high): an aging appearance sagging type

In step 6 (S6), the evaluation device 10 transmits the intrafacial movement induced sag amount $S_f$ and the anteroposterior buccal sag amount $S_c$ that were calculated in S5 to the analysis terminal 30. Note that the evaluation device 10 can be configured to transmit the type of sagging and the degree thereof that were evaluated in S5 to the analysis terminal 30.

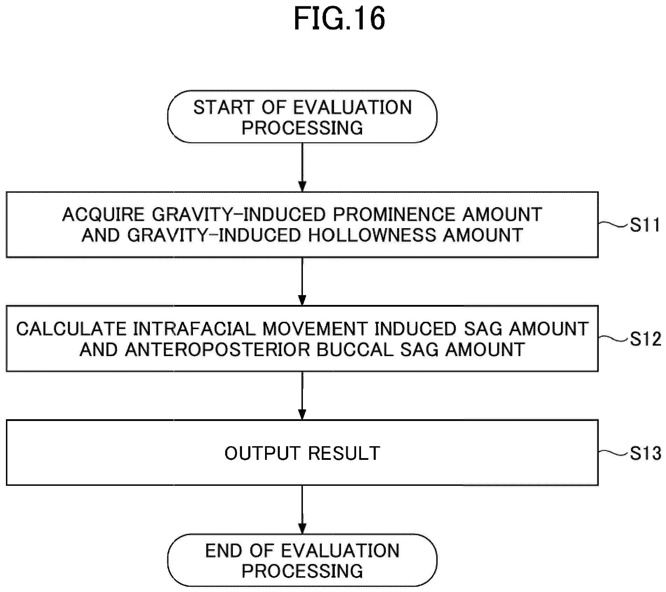

FIG. 16 is a flowchart of the evaluation processing according to one embodiment of the present disclosure. The intrafacial movement induced sag amount $S_f$ and the anteroposterior buccal sag amount $S_c$ are calculated in FIG. 16.

In step 11 (S11), the acquisition unit 101 of the evaluation device 10 acquires the gravity-induced prominence amount $VC_{swelling}$ and the gravity-induced hollowness amount $VC_{shrinking}$. For example, the acquisition unit 101 of the evaluation device 10 acquires the gravity-induced prominence amount $VC_{swelling}$ and the gravity-induced hollowness amount $VC_{shrinking}$ from the analysis terminal 30.

In step 12 (S12), the calculation unit 102 of the evaluation device 10 calculates the intrafacial movement induced sag amount $S_f$ and the anteroposterior buccal sag amount $S_c$ from the gravity-induced prominence amount $VC_{swelling}$ and the gravity-induced hollowness amount $VC_{shrinking}$ acquired in S11. More specifically, the calculation unit 102 of the evaluation device 10 calculates the intrafacial movement induced sag amount $S_f$ and the anteroposterior buccal sag amount $S_c$ from the gravity-induced prominence amount $VC_{swelling}$ and the gravity-induced hollowness amount $VC_{shrinking}$ based on the coordinate conversion by equation (1) described above.

Note that the evaluation unit 103 of the evaluation device 10 can also be configured to evaluate the type of sagging and the degree thereof by using, as indices, the intrafacial movement induced sag amount $S_f$ and the anteroposterior buccal sag amount $S_c$ calculated by the calculation unit 102. For example, the evaluation unit 103 of the evaluation device 10 can evaluate the degree of facial sagging by setting the proportion of the intrafacial movement induced sag amount $S_f$ as the proportion of sagging caused by aging, and setting the proportion of the anteroposterior buccal sag amount $S_c$ as the proportion of sagging caused by individual differences.

In step 13 (S13), the output unit 104 of the evaluation device 10 outputs (for example, transmits to the analysis terminal 30 or displays on the display unit of the evaluation device 10) the intrafacial movement induced sag amount $S_f$ and the anteroposterior buccal sag amount $S_c$ that were calculated in S12. Note that the output unit 104 of the evaluation device 10 can also be configured to output (for example, transmit to the analysis terminal 30 or display on the display unit of the evaluation device 10) the type of sagging and the degree thereof evaluated in S12.

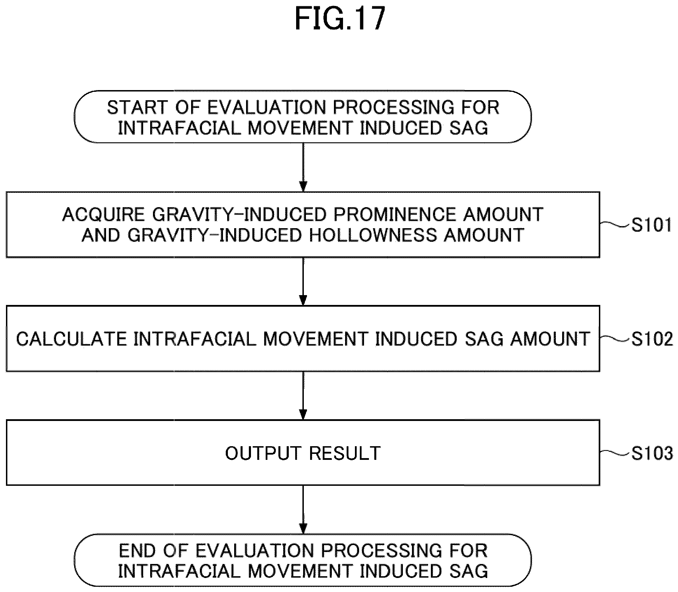

FIG. 17 is a flowchart of the evaluation processing for the intrafacial movement induced sag according to one embodiment of the present disclosure. The intrafacial movement induced sag amount $S_f$ is calculated in FIG. 17.

In step 101 (S101), the acquisition unit 101 of the evaluation device 10 acquires the gravity-induced prominence amount $VC_{swelling}$ and the gravity-induced hollowness amount $VC_{shrinking}$. For example, the acquisition unit 101 of the evaluation device 10 acquires the gravity-induced prominence amount $VC_{swelling}$ and the gravity-induced hollowness amount $VC_{shrinking}$ from the analysis terminal 30.

In step 102 (S102), the calculation unit 102 of the evaluation device 10 calculates the intrafacial movement induced sag amount $S_f$ from the gravity-induced prominence amount $VC_{swelling}$ and the gravity-induced hollowness amount $VC_{shrinking}$ acquired in S101. More specifically, the calculation unit 102 of the evaluation device 10 calculates the intrafacial movement induced sag amount $S_f$ from the gravity-induced prominence amount $VC_{swelling}$ and the gravity-induced hollowness amount $VC_{shrinking}$ based on the coordinate conversion by equation (1) described above.

In step 103 (S103), the output unit 104 of the evaluation device 10 outputs (for example, transmits to the analysis terminal 30 or displays on the display unit of the evaluation device 10) the intrafacial movement induced sag amount $S_f$ calculated in S102.

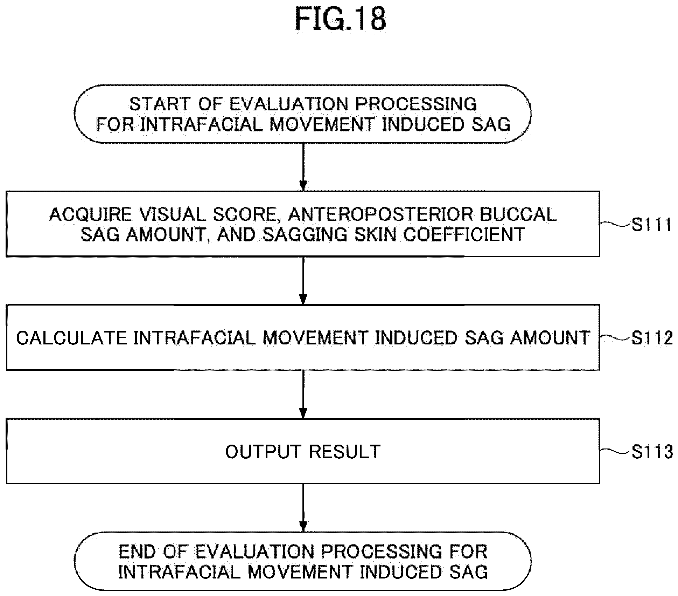

FIG. 18 is a flowchart of the evaluation processing for intrafacial movement induced sag according to one embodiment of the present disclosure. The intrafacial movement induced sag amount $S_f$ is calculated in FIG. 18.

In step 111 (S111), the acquisition unit 101 of the evaluation device 10 acquires the visual score $S_{ss}$, the anteroposterior buccal sag amount $S_c$, and the sagging skin coefficient $C_s$ of the face of the person subject to the sagging evaluation.

For example, the acquisition unit 101 of the evaluation device 10 can acquire the visual score $S_{ss}$ calculated from a facial image acquired when the face of the person subject to sagging evaluation is in the vertical position (that is, in a state where the median plane of the face rests parallel to the direction of gravity). Note that the visual score $S_{ss}$ may be calculated by using a trained model generated by machine learning.

For example, the acquisition unit 101 of the evaluation device 10 can acquire the anteroposterior buccal sag amount $S_c$ calculated from the gravity-induced prominence amount $VC_{swelling}$ and the gravity-induced hollowness amount $VC_{shrinking}$.

For example, the acquisition unit 101 of the evaluation device 10 can acquire the sagging skin coefficient $C_s$ based on skin viscoelasticity that is acquired by a device (viscoelasticity acquisition device) for acquiring skin viscoelasticity.

In step 112 (S1 12), the calculation unit 102 of the evaluation device 10 calculates the intrafacial movement induced sag amount $S_f$ from the visual score $S_{ss}$, the anteroposterior buccal sag amount $S_c$, and the sagging skin coefficient $C_s$ acquired in S111. More specifically, the calculation unit 102 of the evaluation device 10 calculates the intrafacial movement induced sag amount $S_f$ from the visual score $S_{ss}$, the anteroposterior buccal sag amount $S_c$, and the sagging skin coefficient $C_s$ by equation (4) described above.

In step 113 (S113), the output unit 104 of the evaluation device 10 outputs (for example, transmits to the analysis terminal 30 or displays on the display unit of the evaluation device 10) the intrafacial movement induced sag amount $S_f$ calculated in S112.

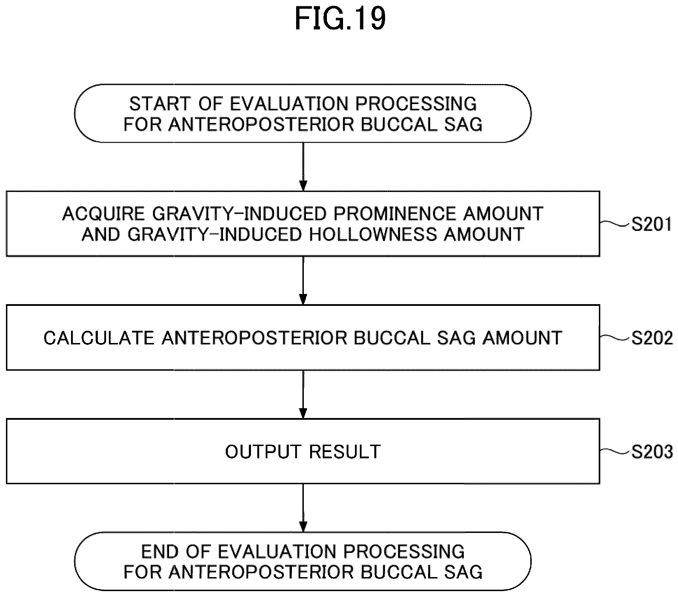

FIG. 19 is a flowchart of the evaluation processing for the anteroposterior buccal sag according to one embodiment of the present disclosure. The anteroposterior buccal sag amount $S_c$ is calculated in FIG. 19.

In step 201 (S201), the acquisition unit 101 of the evaluation device 10 acquires the gravity-induced prominence amount $VC_{swelling}$ and the gravity-induced hollowness amount $VC_{shrinking}$. For example, the acquisition unit 101 of the evaluation device 10 acquires the gravity-induced prominence amount $VC_{swelling}$ and the gravity-induced hollowness amount $VC_{shrinking}$ from the analysis terminal 30.

In step 202 (S202), the calculation unit 102 of the evaluation device 10 calculates the anteroposterior buccal sag amount $S_c$ from the gravity-induced prominence amount $VC_{swelling}$ and the gravity-induced hollowness amount $VC_{shrinking}$ acquired in S201. More specifically, the calculation unit 102 of the evaluation device 10 calculates the anteroposterior buccal sag amount $S_c$ from the gravity-induced prominence amount $VC_{swelling}$ and the gravity-induced hollowness amount $VC_{shrinking}$ based on the coordinate conversion by equation (1) described above.

Note that the evaluation unit 103 of the evaluation device 10 can be configured to evaluate the type of sagging and the degree thereof by using, as an index, the anteroposterior buccal sag amount $S_c$ calculated by the calculation unit 102. For example, the evaluation unit 103 of the evaluation device 10 can evaluate the degree of facial sagging based on an increase or decrease from the anteroposterior buccal sag amount $S_c$ acquired in the past.

In step 203 (S203), the output unit 104 of the evaluation device 10 outputs (for example, transmits to the analysis terminal 30 or displays on the display unit of the evaluation device 10) the anteroposterior buccal sag amount $S_c$ calculated in S202. Note that the output unit 104 of the evaluation device 10 can also be configured to output (for example, transmit to the analysis terminal 30 or display on the display unit of the evaluation device 10) the type of sagging and the degree thereof evaluated in S202.

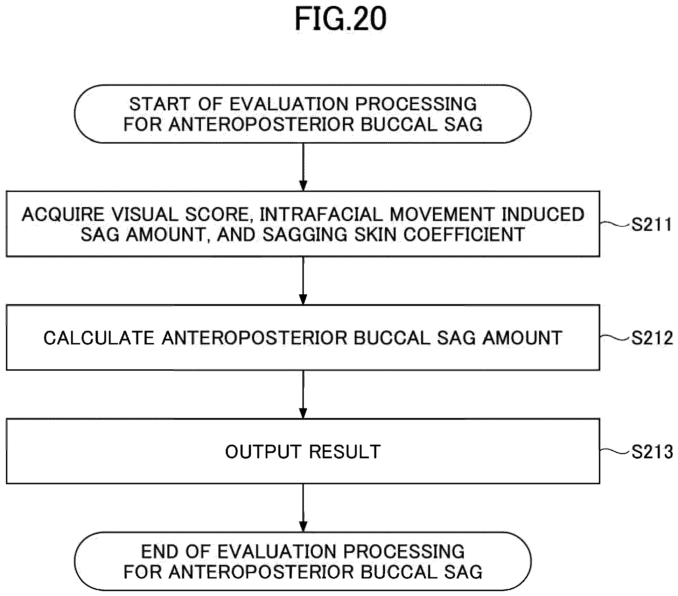

FIG. 20 is a flowchart of the evaluation processing for the anteroposterior buccal sag according to one embodiment of the present disclosure. The anteroposterior buccal sag amount $S_c$ is calculated in FIG. 20.

In step 211 (S211), the acquisition unit 101 of the evaluation device 10 acquires the visual score $S_{ss}$, the intrafacial movement induced sag amount $S_f$, and the sagging skin coefficient $C_s$ of the face of the person subject to sagging evaluation.

For example, the acquisition unit 101 of the evaluation device 10 can acquire the visual score $S_{ss}$ calculated from a facial image acquired when the face of the person subject to sagging evaluation is in the vertical position (that is, in a state where the median plane of the face rests parallel to the direction of gravity). Note that the visual score $S_{ss}$ may be calculated by using a trained model generated by machine learning.

For example, the acquisition unit 101 of the evaluation device 10 can acquire the intrafacial movement induced sag amount $S_f$ calculated from the gravity-induced prominence amount $VC_{swelling}$ and the gravity-induced hollowness amount $VC_{shrinking}$.

For example, the acquisition unit 101 of the evaluation device 10 can acquire the sagging skin coefficient $C_s$ based on skin viscoelasticity that is acquired by a device (viscoelasticity acquisition device) for acquiring skin viscoelasticity.

In step 212 (S212), the calculation unit 102 of the evaluation device 10 calculates the anteroposterior buccal sag amount $S_c$ from the visual score $S_{ss}$, the intrafacial movement induced sag amount $S_f$, and the sagging skin coefficient $C_s$ acquired in S211. More specifically, the calculation unit 102 of the evaluation device 10 calculates the anteroposterior buccal sag amount $S_c$ from the visual score $S_{ss}$, the intrafacial movement induced sag amount $S_f$, and the sagging skin coefficient $C_s$ by equation (4) described above.

Note that the evaluation unit 103 of the evaluation device 10 can be configured to evaluate the type of sagging and the degree thereof by using, as an index, the anteroposterior buccal sag amount $S_c$ calculated by the calculation unit 102. For example, the evaluation unit 103 of the evaluation device 10 can evaluate the degree of facial sagging based on an increase or a decrease from the anteroposterior buccal sag amount $S_c$ acquired in the past.

In step 213 (S213), the output unit 104 of the evaluation device 10 outputs (for example, transmits to the analysis terminal 30 or displays on the display unit of the evaluation device 10) the anteroposterior buccal sag amount $S_c$ calculated in S212. Note that the output unit 104 of the evaluation device 10 can also be configured to output (for example, transmit to the analysis terminal 30 or display on the display unit of the evaluation device 10) the type of sagging and it degree thereof evaluated in S212.

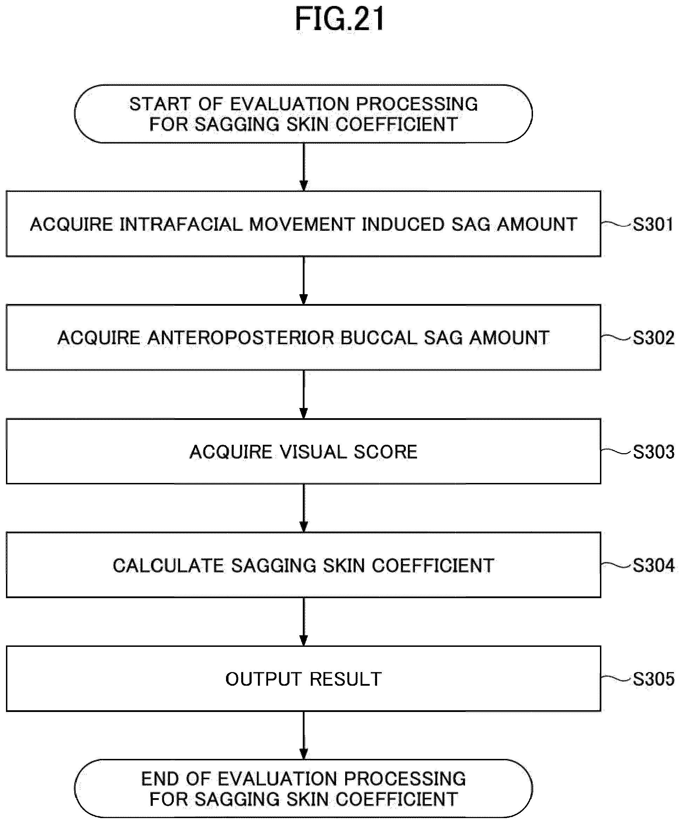

FIG. 21 is a flowchart of the evaluation processing for the sagging skin coefficient according to one embodiment of the present disclosure. The sagging skin coefficient $C_s$ is calculated in FIG. 21. The sagging skin coefficient $C_s$ can be directly calculated from the skin viscoelasticity acquired by the viscoelasticity acquisition device by equation (5) or (6) described above or be calculated by equation (2) described above.

In step 301 (S301), the acquisition unit 101 of the evaluation device 10 acquires the intrafacial movement induced sag amount $S_\varepsilon$. For example, the acquisition unit 101 of the evaluation device 10 can acquire the intrafacial movement induced sag amount $S_f$ calculated from the gravity-induced prominence amount $VC_{swelling}$ and the gravity-induced hollowness amount $VC_{shrinking}$.

In step 302 (S302), the acquisition unit 101 of the evaluation device 10 acquires the anteroposterior buccal sag amount $S_c$. For example, the acquisition unit 101 of the evaluation device 10 can acquire the anteroposterior buccal sag amount $S_c$ calculated from the gravity-induced prominence amount $VC_{swelling}$ and the gravity-induced hollowness amount $VC_{shrinking}$.

In step 303 (S303), the acquisition unit 101 of the evaluation device 10 acquires the visual score $S_{ss}$. For example, the acquisition unit 101 of the evaluation device 10 can acquire the visual score $S_{ss}$ calculated from a facial image acquired when the face of the person subject to sagging evaluation is in the vertical position (that is, in a state where the median plane of the face rests parallel to the direction of gravity). Note that the visual score $S_{ss}$ may be calculated by using a trained model generated by machine learning.

Note that S301 to S303 may be performed in any order and may be performed simultaneously.

In step 304 (S304), the calculation unit 102 of the evaluation device 10 calculates the sagging skin coefficient $C_s$ from the intrafacial movement induced sag amount $S_f$, the anteroposterior buccal sag amount $S_c$, and the visual score $S_{ss}$ acquired in S301 to S303. More specifically, the calculation unit 102 of the evaluation device 10 calculates the sagging skin coefficient $C_s$ from the intrafacial movement induced sag amount $S_f$, the anteroposterior buccal sag amount $S_c$, and the visual score $S_{ss}$ by equation (4) described above.

Note that the evaluation unit 103 of the evaluation device 10 can also be configured to evaluate the type of sagging and the degree thereof by using, as an index, the sagging skin coefficient $C_s$ calculated by the calculation unit 102. For example, the evaluation unit 103 of the evaluation device 10 can evaluate the degree of facial sagging based on an increase or a decrease from the sagging skin coefficient $C_s$ acquired in the past. The degree of facial sagging may also be evaluated by whether the subject's sagging skin coefficient is greater or less than the average of his or her real age.

In step 305 (S305), the output unit 104 of the evaluation device 10 outputs (for example, transmits to the analysis terminal 30 or displays on the display unit of the evaluation device 10) the sagging skin coefficient $C_s$ calculated in S304. Note that the output unit 104 of the evaluation device 10 can also be configured to output (for example, transmit to the analysis terminal 30 or display on the display unit of the evaluation device 10) the type of sagging and the degree thereof evaluated in S304.

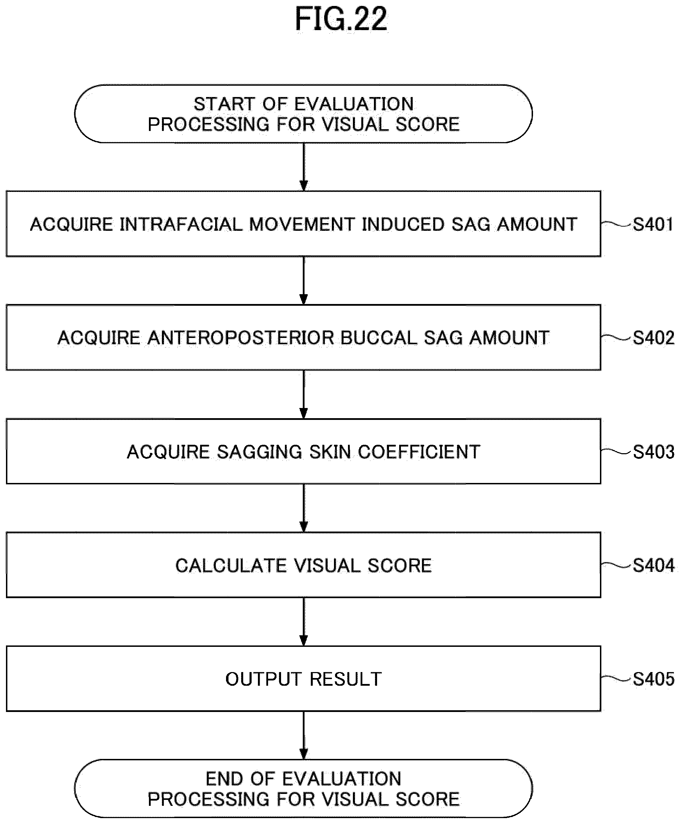

FIG. 22 is a flowchart of the evaluation processing for the visual score according to one embodiment of the present disclosure. The visual score $S_{ss}$ is calculated in FIG. 22.

In step 401 (S401), the acquisition unit 101 of the evaluation device 10 acquires the intrafacial movement induced sag amount $S_f$. For example, the acquisition unit 101 of the evaluation device 10 can acquire the intrafacial movement induced sag amount $S_f$ calculated from the gravity-induced prominence amount $VC_{swelling}$ and the gravity-induced hollowness amount $VC_{shrinking}$.

In step 402 (S402), the acquisition unit 101 of the evaluation device 10 acquires the anteroposterior buccal sag amount $S_c$. For example, the acquisition unit 101 of the evaluation device 10 can acquire the anteroposterior buccal sag amount $S_c$ calculated from the gravity-induced prominence amount $VC_{swelling}$ and the gravity-induced hollowness amount $VC_{shrinking}$.

In step 403 (S403), the acquisition unit 101 of the evaluation device 10 acquires the sagging skin coefficient $C_s$. For example, the acquisition unit 101 of the evaluation device 10 can acquire the sagging skin coefficient $C_s$ based on skin viscoelasticity that is acquired by a device (viscoelasticity acquisition device) for acquiring skin viscoelasticity.

Note that S401 to S403 may be performed in any order and may be performed simultaneously.

In step 404 (S404), the calculation unit 102 of the evaluation device 10 calculates the visual score $S_{ss}$ from the intrafacial movement induced sag amount $S_f$, the anteroposterior buccal sag amount $S_c$, and the sagging skin coefficient $C_s$ acquired in S401 to S403. More specifically, the calculation unit 102 of the evaluation device 10 calculates the visual score $S_{ss}$ from the intrafacial movement induced sag amount $S_f$, the anteroposterior buccal sag amount $S_c$, and the sagging skin coefficient $C_s$ by equation (4) described above.

In step 405 (S405), the output unit 104 of the evaluation device 10 outputs (for example, transmits to the analysis terminal 30 or displays on the display unit of the evaluation device 10) the visual score $S_{ss}$ calculated in S404.

«Recommendation of Solution to Sagging»

In the sagging evaluation method described above, it is also possible to output a solution to sagging along with the evaluation result (each of the values, the type of sagging, and the degree thereof). More specifically, the output unit 104 of the evaluation device 10 outputs a predetermined solution corresponding to the evaluation result (each of the values, the type of sagging, and the degree thereof). For example, in a case where there is a high proportion of the intrafacial movement induced sag amount $S_f$, the output unit 104 of the evaluation device 10 recommends cosmetic treatments and training that include cosmetics, supplements, and massages for resolving the intrafacial movement induced sag. In a case where there is a high proportion of the anteroposterior buccal sag amount $S_c$, the output unit 104 of the evaluation device 10 recommends cosmetic treatments and training that include cosmetics, supplements, and massages for resolving the anteroposterior buccal sag. For example, the output unit 104 of the evaluation device 10 recommends cosmetic treatments and training that include cosmetics, supplements, and massages corresponding to the intrafacial movement induced sag amount $S_f$ and the anteroposterior buccal sag amount $S_c$.

<<Weighting>>

In the evaluation of the type of sagging and the degree thereof described above, the evaluation of factors may be weighted based on the rate of contribution of the sagging skin coefficient $C_s$ with respect to the visual score $S_{ss}$, the rate of contribution of the intrafacial movement induced sag amount $S_f$ with respect to the visual score $S_{ss}$, and the rate of contribution of the anteroposterior buccal sag amount $S_c$ with respect to the visual score $S_{ss}$.

«Iterative Acquisition of Three-dimensional Face Image»

In one embodiment of the present disclosure, three-dimensional face images of a subject in the horizontal position and the vertical position are acquired iteratively, and at least one of the gravity-induced prominence amount $VC_{swelling}$, the gravity-induced hollowness amount $VC_{shrinking}$, the intrafacial movement induced sag amount $S_f$, or the anteroposterior buccal sag amount $S_c$ can be set as a representative value of values calculated by using the three-dimensional face images that are iteratively acquired. This will be described in detail below.

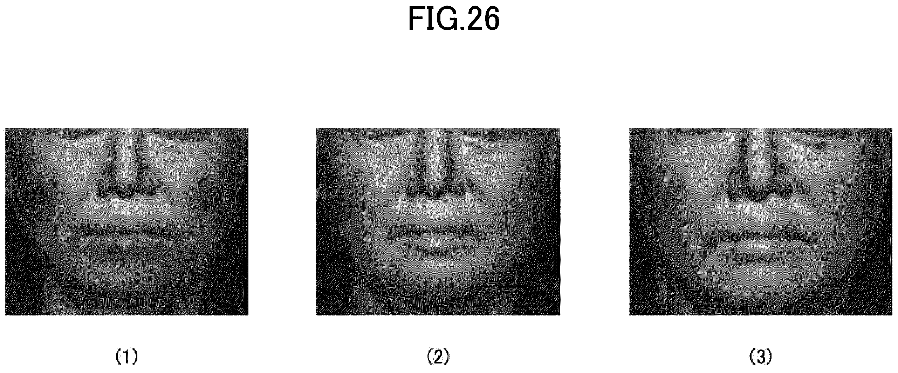

FIG. 26 is a view illustrating three-dimensional face images acquired in the same period of time, on the same date, and in the same place. The 3 three-dimensional face images (1), (2), and (3) in FIG. 26 are three-dimensional face images of the same person captured in the same period of time, on the same date, in the same place. As illustrated in FIG. 26, it can be seen that the facial shape varies even on the same date, in the same place, and in the same period of time, and that there is movement particularly around the mouth.

That is, even when three-dimensional face images of the same person are acquired on the same date, in the same place, and in the same period of time, there is variation in the gravity-induced prominence amount $VC_{swelling}$ and the gravity-induced hollowness amount $VC_{shrinking}$.

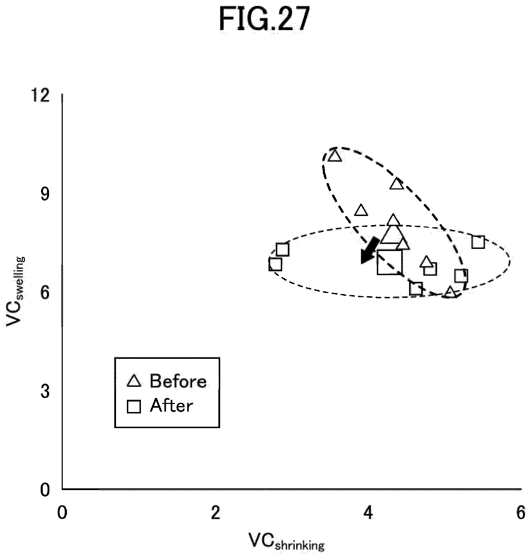

FIG. 27 is a graph illustrating the variation in the gravity-induced prominence and the gravity-induced hollowness according to one embodiment of the present disclosure. Each triangle in FIG. 27 indicates the gravity-induced prominence amount $VC_{swelling}$ and the gravity-induced hollowness amount $VC_{shrinking}$ of a given subject before the subject was treated by a solution (specifically, a massage to the scalp) for sagging. Each square in FIG. 27 indicates the gravity-induced prominence amount $VC_{swelling}$ and the gravity-induced hollowness amount $VC_{shrinking}$ of the subject after the

EP 4 160 525 A1

subject was treated by the solution (specifically, a massage to the scalp) for sagging. As it can be seen in FIG. 27, the gravity-induced prominence amount $VC_{swelling}$ and the gravity-induced hollowness amount $VC_{shrinking}$ vary both before and after the subject was treated by the solution for sagging. Hence, if three-dimensional face images of the subject in the horizontal position and the vertical position are not acquired iteratively, it may result in an evaluation that there is more sagging after the subject was treated by the solution for sagging than there was before the subject was treated by the solution for sagging. Note that the average value of the gravity-induced prominence amount $VC_{swelling}$ is represented by a large triangle and the average value of the gravity-induced hollowness amount $VC_{shrinking}$ is represented by a large square.

Hence, in one embodiment of the present disclosure, three-dimensional face images of the subject in the horizontal position and the vertical position are acquired iteratively, and at least one of the gravity-induced prominence amount $VC_{swelling}$, the gravity-induced hollowness amount $VC_{shrinking}$, the intrafacial movement induced sag amount $S\varepsilon$, or the anteroposterior buccal sag amount $S_c$ can be set as a representative value of values calculated by using the three-dimensional face images that are iteratively acquired.

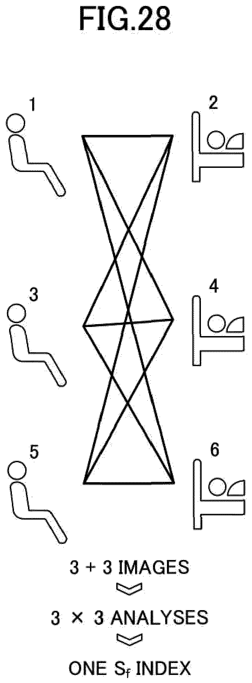

FIG. 28 is a view for explaining the iterative acquisition of three-dimensional face images according to one embodiment of the present disclosure. The case of the intrafacial movement induced sag amount $S\varepsilon$ will be described hereinafter. The evaluation device 10 iteratively acquires the three-dimensional face images of the subject in the horizontal position and the vertical position (acquires a total of 6 images, that is, 3 three-dimensional face images in the horizontal position and 3 three-dimensional face images in the vertical position, in the example of FIG. 28). Subsequently, the evaluation device 10 acquires the gravity-induced prominence amount $VC_{swelling}$ and the gravity-induced hollowness amount $VC_{shrinking}$ from the changes between the three-dimensional shapes of the three-dimensional face images of the iteratively acquired three-dimensional face images. For example, in FIG. 28, 9 pairs of the gravity-induced prominence amount $VC_{swelling}$ and the gravity-induced hollowness amount $VC_{shrinking}$ are acquired from 9 combinations of three-dimensional face images obtained by combining the 3 three-dimensional face images in the horizontal position and the 3 three-dimensional face images in the vertical position. The evaluation device 10 subsequently calculates the representative value of the intrafacial movement induced sag amount $S\varepsilon$, which is calculated from pairs of the gravity-induced prominence amount $VC_{swelling}$ and the gravity-induced hollowness amount $VC_{shrinking}$, as the intrafacial movement induced sag amount $S_f$. Note that the representative value is, for example, the average value, but may also be the median or the mode.

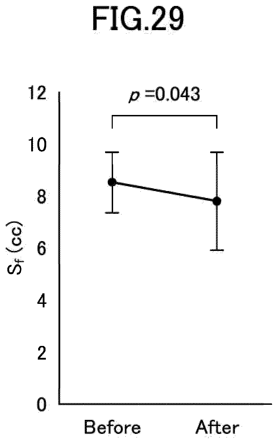

FIG. 29 is a graph for explaining the representative values of at least one of the gravity-induced prominence amount $VC_{swelling}$, the gravity-induced hollowness amount $VC_{shrinking}$, the intrafacial movement induced sag amount $S_f$, or the anteroposterior buccal sag amount $S_c$ based on values calculated by using the iteratively acquired three-dimensional face images according to one embodiment of the present disclosure. The case of the intrafacial movement induced sag amount $S\varepsilon$ will be described hereinafter. The average value of 9 intrafacial movement induced sag amounts $S_f$, which were calculated from 9 pairs of the gravity-induced prominence amount $VC_{swelling}$ and the gravity-induced hollowness amount $VC_{shrinking}$ acquired before the given subject was treated by the solution (specifically, a massage to the scalp) for sagging indicated in FIG. 27, is compared with the average value of 9 intrafacial movement induced sag amounts $S_f$, which were calculated from 9 pairs of the gravity-induced prominence amount $VC_{swelling}$ and the gravity-induced hollowness amount $VC_{shrinking}$ acquired after the given subject was treated by the solution (specifically, a massage to the scalp) for sagging. It can be seen that the treatment by the solution (specifically, a massage to the scalp) for sagging has resulted in a slight but significant reduction in the intrafacial movement induced sag amount $S_f$.

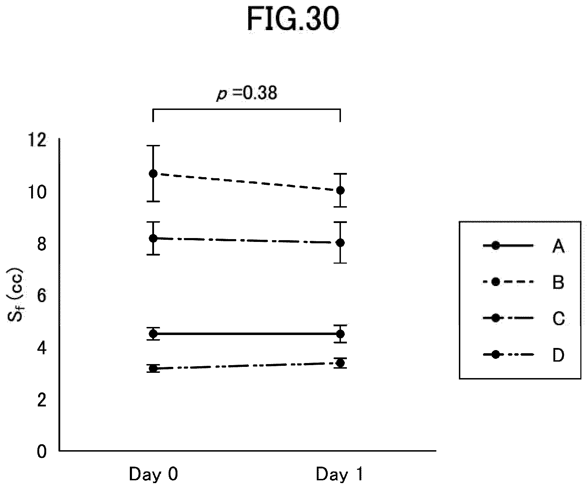

FIG. 30 is a graph for explaining daily differences between the representative values of at least one of the gravity-induced prominence amount $VC_{swelling}$, the gravity-induced hollowness amount $VC_{shrinking}$, the intrafacial movement induced sag amount $S_f$, or the anteroposterior buccal sag amount $S_c$ based on values calculated by using the three-dimensional face images that were acquired iteratively according to one embodiment of the present disclosure. The case of the intrafacial movement induced sag amount $S\varepsilon$ will be described hereinafter. In FIG. 30, the average value of 9 intrafacial movement induced sag amounts $S_f$ calculated from 9 pairs of the gravity-induced prominence amount $VC_{swelling}$ and the gravity-induced hollowness amount $VC_{shrinking}$ was acquired for each of the four subjects for a given day and the day after. No treatments (such as use of facial care devices or massages to the scalp) that could affect the sagging were performed during this period. Hence, it was expected that there would be no change in the sagging for each subject, and that there would be no significant difference in the average value of intrafacial movement induced sag amount $S_f$ of each subject. Since there is actually no significant difference in all of the intrafacial movement induced sag amounts $S_f$ of the four subjects, it is possible to follow the individual changes in sagging for each subject. Furthermore, since $p = 0.38$, there is also no significant difference in the daily differences among the average values of the intrafacial movement induced sag amounts $S_f$ of the four subjects. Thus, for the purpose of measuring the effects on sagging, subjects or groups of subjects who have undergone treatment can be compared with those who have not, or subjects or groups of subjects who have undergone treatments of different strengths can be compared.

FIG. 31 is a graph for explaining a result of comparing subjects or groups of subjects who have undergone different strengths of treatments or comparing those who have undergone treatment with those who have not for the purpose of measuring the effect of the treatments on sagging by acquiring the representative values of least one of the gravity-induced prominence amount $VC_{swelling}$, the gravity-induced hollowness amount $VC_{shrinking}$, the intrafacial movement induced sag amount $S_f$, or the anteroposterior buccal sag amount $S_c$ based on values calculated by using the three-dimensional face images that were acquired iteratively. The case of the intrafacial movement induced sag amount $S_f$ will be described hereinafter. In FIG. 31, for the purpose of measuring the effect on sagging, treatments by a commercial facial care device (Denki Bari Brush from ELECTRON EVERYONE CO.) were performed on two subjects, and the average values of the intrafacial movement induced sag amounts $S_f$ calculated by using three-dimensional face images the iteratively acquired immediately before the treatment, immediately after the treatment, and the day after the treatment, and the average values were compared. For subject E, no significant differences were observed among all of the intrafacial movement induced sag amounts $S_f$ calculated by using the three-dimensional face images iteratively acquired immediately before the treatment, immediately after the treatment, and the day after the treatment. This result represents that the treatment by the commercial facial care device (Denki Bari Brush from ELECTRON EVERYONE CO.), which was performed for the purpose of measuring effect on sagging, had no effect for subject E. For subject F, no significant differences were observed between the intrafacial movement induced sag amounts $S_f$ calculated by using the three-dimensional face images iteratively acquired immediately before the treatment and the day after the treatment. However, significant differences were observed between the intrafacial movement induced sag amounts $S_f$ calculated by using the three-dimensional face images iteratively acquired immediately before the treatment and immediately after the treatment, and significant differences were observed between the intrafacial movement induced sag amounts $S_f$ calculated by using the three-dimensional face images iteratively acquired immediately after the treatment and the day after the treatment. This result represents that, for subject F, the treatment by the commercial facial care device (Denki Bari Brush from ELECTRON EVERYONE CO.), which was performed for the purpose of measuring effect on sagging, had an adverse effect on sagging immediately after the treatment, but the effect dissipated the day after the treatment and the sagging returned to the original state.

Note that one embodiment of the present disclosure can further include selecting substances or cosmetic treatments that increase or decrease the representative value of the values calculated by using the iteratively acquired three-dimensional face images. In this manner, one embodiment of the present disclosure can select and acquire substances or cosmetic treatments that increase or decrease the representative value of the values calculated by using the iteratively acquired three-dimensional face images. For example, the substances are materials, such as plant extracts or compounds, intended to be mixed into cosmetics. Also, the substances are, for example, cosmetics, drugs, food, or the like. Furthermore, one embodiment of the present disclosure can further include presenting a cosmetic method for increasing the representative value of the values calculated by using the iteratively acquired three-dimensional face images, based on the representative value of the values calculated by using the iteratively acquired three-dimensional face images.

<Hardware Configuration>

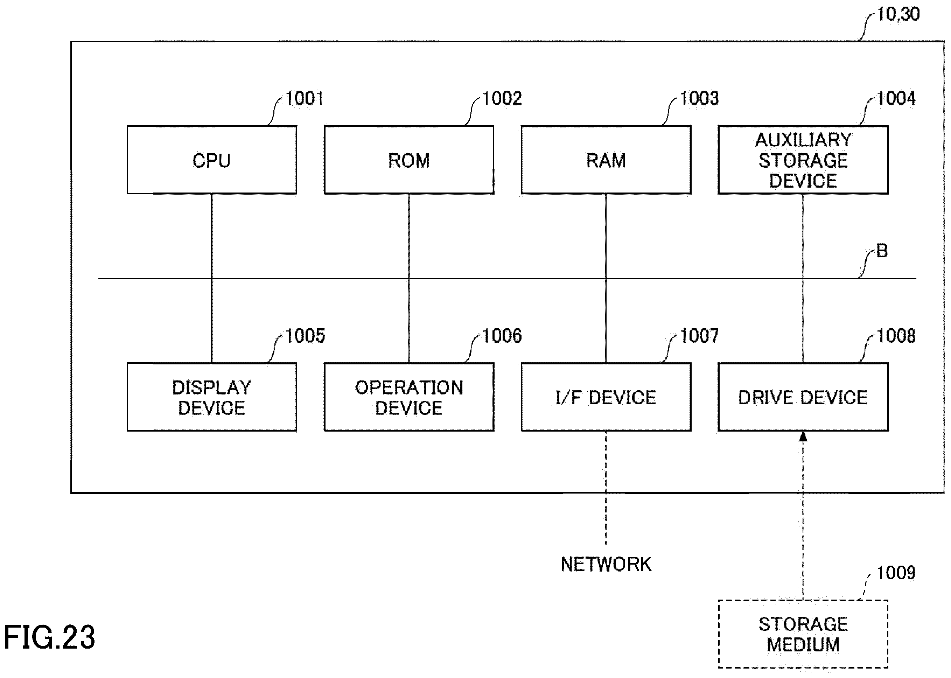

FIG. 23 is a block diagram illustrating an example of a hardware configuration of the information processing system 10 and the analysis terminal 30 according to one embodiment of the present disclosure. The information processing system 10 and the analysis terminal 30 each include a central processing unit (CPU) 1001, a read-only memory(ROM) 1002, and a random access memory (RAM) 1003. The CPU 1001, the ROM 1002, and the RAM 1003 form a computer.

The evaluation device 10 and the analysis terminal 30 each can include an auxiliary storage device 1004, a display device 1005, an operation device 1006, an I/F (interface) device 1007, and a drive device 1008. Each piece of the hardware of the evaluation device 10 and the analysis terminal 30 is connected to each other via a bus B.

The CPU 1001 is an arithmetic device that executes various programs installed in the auxiliary storage device 1004.

The ROM 1002 is a non-volatile memory. The ROM 1002 functions as a main storage device that stores various programs and data necessary for executing various programs installed in the auxiliary storage device 1004 by the CPU 1001. Specifically, the ROM 1002 functions as a main storage device that stores boot programs such as a basic input/output system (BIOS) and an extensible firmware interface (EFI).

The RAM 1003 is a volatile memory such as a dynamic random access memory (DRAM) and a static random access memory (SRAM). The RAM 1003 functions as a main storage device that provides a workspace deployed when various programs installed in the auxiliary storage device 1004 are executed by the CPU 1001.

The auxiliary storage device 1004 is an auxiliary storage device that stores various programs or information to be used when the various programs are executed.

The display device 1005 is a display device that displays an internal state or the like of the evaluation device 10 and the analysis terminal 30.

The operation device 1006 is an input device in which an administrator of the evaluation device 10 and the analysis terminal 30 inputs various instructions to the evaluation device 10 and the analysis terminal 30.

The I/F device 1007 is a communication device for connecting to a network and communicating with other devices.

The drive device 1008 is a device for setting the storage medium 1009. The storage medium 1009 herein includes a medium for optically, electrically, or magnetically recording information, such as a CD-ROM, a flexible disk, or a magneto-optical disk. Further, the storage medium 1009 may include a semiconductor memory or the like that electrically records information, such as an erasable programmable read-only memory (EPROM), a flash memory, or the like.

Note that the various programs installed in the auxiliary storage device 1004 are installed when, for example, the distributed storage medium 1009 is set in the drive device 1008 and various programs recorded in the storage medium 1009 are read out by the drive device 1008. Alternatively, various programs installed in the auxiliary storage device 1004 may be installed by being downloaded from the network via the I/F device 1007.

Although embodiments have been described in detail above, the present disclosure is not limited to the specific embodiments described above, and various modifications and substitutions can be made without departing from the scope of the claims.

This international application claims priority based on Japanese Patent Application No. 2020-092470 filed on May 27, 2020 and on Japanese Patent Application No. 2020-209513 filed on December 17, 2020. The entire contents of Japanese Patent Application No. 2020-092470 and Japanese Patent Application No. 2020-209513 are incorporated herein by reference.

**[REFERENCE SYMBOLS LIST]**

| | |
|---|---|
| 1 | evaluation system |
| 10 | evaluation device |
| 20 | image capturing terminal |
| 30 | analysis terminal |
| 101 | acquisition unit |
| 102 | calculation unit |
| 103 | evaluation unit |
| 104 | output unit |
| 201 | generation unit |
| 301 | calculation unit |
| 1001 | CPU |
| 1002 | ROM |
| 1003 | RAM |
| 1004 | auxiliary storage device |
| 1005 | display device |
| 1006 | operation device |
| 1007 | I/F device |
| 1008 | drive device |
| 1009 | storage medium |

**Claims**

1. A sagging evaluation method comprising:

   a step of acquiring a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position;
   a step of calculating an intrafacial movement induced sag amount and an anteroposterior buccal sag amount from the gravity-induced prominence amount and the gravity-induced hollowness amount; and
   a step of evaluating a type of sagging and a degree thereof based on the intrafacial movement induced sag amount and the anteroposterior buccal sag amount as indices.

2. The sagging evaluation method according to claim 1, wherein letting $VC_{swelling}$ be the gravity-induced prominence amount, $VC_{shrinking}$ be the gravity-induced hollowness amount, $S_f$ be the intrafacial movement induced sag amount, and $S_c$ be the anteroposterior buccal sag amount, the intrafacial movement induced sag amount and the anteroposterior buccal sag amount are obtained as follows:

$$\begin{pmatrix} S_f \\ S_c \end{pmatrix} = \begin{pmatrix} \cos\theta & \sin\theta \\ -\sin\theta & \cos\theta \end{pmatrix} \begin{pmatrix} VC_{shrinking} \\ VC_{swelling} \end{pmatrix} \quad \cdots (1)$$

where $VC_{swelling}$ is the gravity-induced prominence amount, $VC_{shrinking}$ is the gravity-induced hollowness amount, $S_f$ is the intrafacial movement induced sag amount, $S_c$ is the anteroposterior buccal sag amount, and $\theta$ is $\pi/4$.

3. The sagging evaluation method according to claim 2, further comprising a step of acquiring a visual score from a facial image of the subject in the vertical position and calculating a sagging skin coefficient from the visual score by

$$S_{ss} = C_s \times \frac{1}{\sqrt{2}}(S_c + S_f) \quad \cdots (4)$$

where $S_{ss}$ is the visual score, $S_f$ is the intrafacial movement induced sag amount, $S_c$ is the anteroposterior buccal sag amount, and $C_s$ is the sagging skin coefficient.

4. The sagging evaluation method according to claim 2, further comprising a step of acquiring a sagging skin coefficient based on skin viscoelasticity of the subject acquired by a viscoelasticity acquisition device and calculating a visual score from the sagging skin coefficient by

$$S_{ss} = C_s \times \frac{1}{\sqrt{2}}(S_c + S_f) \quad \cdots (4)$$

where $S_{ss}$ is the visual score, $S_f$ is the intrafacial movement induced sag amount, $S_c$ is the anteroposterior buccal sag amount, and $C_s$ is the sagging skin coefficient.

5. The sagging evaluation method according to any one of claims 1 to 4, further comprising a step of iteratively acquiring the three-dimensional face images of the subject in the horizontal position and the three-dimensional face images of the subject in the vertical position,
wherein at least one of the gravity-induced prominence amount, the gravity-induced hollowness amount, the intrafacial movement induced sag amount, or the anteroposterior buccal sag amount is a representative value of values calculated by using the iteratively acquired three-dimensional face images.

6. The sagging evaluation method according to any one of claims 1 to 4, further comprising a step of iteratively acquiring the three-dimensional face images of the subject in the horizontal position and the three-dimensional face images of the subject in the vertical position,

wherein the gravity-induced prominence amount and the gravity-induced hollowness amount are acquired from the change between the three-dimensional shapes of the three-dimensional face images of each of the iteratively acquired three-dimensional face images, and
wherein the step of calculating the intrafacial movement induced sag amount includes calculating, as the intrafacial movement induced sag amount, a representative value of the intrafacial movement induced sag amounts, which are calculated from the intrafacial movement induced sag amounts and the gravity-induced prominence amounts of the iteratively acquired three-dimensional face images.

7. The sagging evaluation method according to claim 5 or 6, further comprising selecting a substance or a cosmetic treatment that increases or reduces the representative value of the values calculated by using the iteratively acquired three-dimensional face images.

8. The substance or the cosmetic treatment selected by the sagging evaluation method according to claim 7.

9. The sagging evaluation method according to claim 5 or 6, further comprising presenting a cosmetic method that increases the representative value of the values calculated by using the iteratively acquired three-dimensional face images, the cosmetic method being presented based on the representative value of the values calculated by using the iteratively acquired three-dimensional face images.

10. A device for evaluating a type of sagging and a degree thereof, comprising:

an acquisition unit configured to acquire a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position;
a calculation unit configured to calculate an intrafacial movement induced sag amount and an anteroposterior buccal sag amount from the gravity-induced prominence amount and the gravity-induced hollowness amount; and
an output unit configured to output the intrafacial movement induced sag amount and the anteroposterior buccal sag amount.

11. A method executed by a device for evaluating a type of sagging and a degree thereof, the method comprising:

a step of acquiring a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position;
a step of calculating an intrafacial movement induced sag amount and an anteroposterior buccal sag amount from the gravity-induced prominence amount and the gravity-induced hollowness amount; and
a step of outputting the intrafacial movement induced sag amount and the anteroposterior buccal sag amount.

12. A program for causing a device for evaluating a type of sagging and a degree thereof to execute processing to

acquire a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position;
calculate an intrafacial movement induced sag amount and an anteroposterior buccal sag amount from the gravity-induced prominence amount and the gravity-induced hollowness amount; and
output the intrafacial movement induced sag amount and the anteroposterior buccal sag amount.

13. A system for evaluating a type of sagging and a degree thereof, the system comprising an image capturing terminal, an analysis terminal, and a server,

wherein the image capturing terminal is configured to generate data of a three-dimensional face image of a subject in a horizontal position and a three-dimensional shape of a three-dimensional face image of the subject in a vertical position,
wherein the analysis terminal is configured to calculate, from the data, a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position, and
wherein the server includes

an acquisition unit configured to acquire the gravity-induced prominence amount and the gravity-induced hollowness amount,
a calculation unit configured to calculate an intrafacial movement induced sag amount and an anteroposterior buccal sag amount from the gravity-induced prominence amount and the gravity-induced hollowness amount, and
an output unit configured to output the intrafacial movement induced sag amount and the anteroposterior buccal sag amount.

**14.** A method comprising:

a step of evaluating three of a visual score on sagging of a face, an intrafacial movement induced sag amount of the face, an anteroposterior buccal sag amount of the face, or a sagging skin coefficient of the face; and
a step of evaluating, based on the three evaluations, one other of the three of the visual score on sagging of the face, the intrafacial movement induced sag amount of the face, the anteroposterior buccal sag amount of the face, or the sagging skin coefficient of the face.

**15.** A method of evaluating a type of sagging and a degree thereof, comprising:

a step of acquiring a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position;
a step of calculating an anteroposterior buccal sag amount from the gravity-induced prominence amount and the gravity-induced hollowness amount; and
a step of evaluating the type of sagging and the degree thereof based on the anteroposterior buccal sag amount as an index.

**16.** A method of evaluating a type of sagging and a degree thereof, comprising:

a step of acquiring a gravity-induced prominence amount and a gravity-induced hollowness amount that are acquired from a change between a three-dimensional shape of a three-dimensional face image of a subject in a horizontal position and a three-dimensional shape of a three-dimensional face image of the subject in a vertical position;
a step of calculating an intrafacial movement induced sag amount from the gravity-induced prominence amount and the gravity-induced hollowness amount; and
a step of evaluating the type of sagging and the degree thereof based on the intrafacial movement induced sag amount as an index.

**17.** A method of evaluating a type of sagging and a degree thereof, comprising:

a step of acquiring a gravity-induced prominence amount that is acquired from a change between a three-dimensional shape of a three-dimensional face image of a subject in a horizontal position and a three-dimensional shape of a three-dimensional face image of the subject in a vertical position;
a step of acquiring a visual score from a facial image of the subject in the vertical position;
a step of calculating a sagging skin coefficient from the gravity-induced prominence amount and the visual score by

$$S_{ss} = C_s \times VC_{swelling} \quad \cdots (2)$$

where $VC_{swelling}$ is the gravity-induced prominence amount, $S_{ss}$ is the visual score, and $C_s$ is the sagging skin coefficient; and
a step of evaluating the type of sagging and the degree thereof based on the sagging skin coefficient as an index.

**18.** A method of evaluating a type of sagging and a degree thereof, comprising:

a step of acquiring a sagging skin coefficient based on a skin viscoelasticity of a subject acquired by a viscoelasticity acquisition device; and
a step of evaluating the type of sagging and the degree thereof based on the sagging skin coefficient as an index.

FIG.1

FIG.2

# FIG.3

# FIG.4

FIG.5

# FIG.6

| | INTRAFACIAL MOVEMENT INDUCED SAG ($S_f$) | ANTEROPOSTERIOR BUCCAL SAG ($S_c$) |
|---|---|---|
| SITE OF OCCURRENCE | ENTIRE CHEEK | LOCALLY AROUND MOUTH AREA |
| RATE OF CONTRIBUTION TO VISUAL SCORE | 53% | 9% (GROUP WITH ANTEROPOSTERIOR BUCCAL SAG)<br><br>0.4% (GROUP WITHOUT ANTEROPOSTERIOR BUCCAL SAG) |
| CORRELATION WITH AGE | 0.64 | <0.10 |
| AMOUNT OF CHANGE EQUAL TO VISUAL SCORE OF 0.5 AND ITS MEANING | 1.3 cc | 1.8 cc |
| | CORRESPONDING TO AGING BY ADDITIONAL 14 YEARS | PRESENCE OR ABSENCE OF ANTEROPOSTERIOR BUCCAL SAG |
| CHARACTERISTICS OF EACH FACTOR | GRAVITY-INDUCED SAGGING THAT GRADUALLY ADVANCES THROUGHOUT ENTIRE FACE LONG TERM | SAGGING THAT OCCURS LOCALLY AROUND MOUTH AREA REGARDLESS OF AGE |

EP 4 160 525 A1

# FIG.7

# FIG.8

R0: Uf(Total elongation)
R1: Uf−Ua
R2: Ua/Uf
R3: last max. amplitude
R4: last min. amplitude
R5: Ur/Ue(Net elasticity)
R6: Uv/Ue(Viscoelasticity)
R7: Ur/Uf(Skin firmness)
R8: Ua(Total recovery)

| $C_s$ | R0 | R1 | R2 | R3 | R4 | R5 | R6 | R7 | R8 | R9 | Ue | Uv | Ur |
|---|---|---|---|---|---|---|---|---|---|---|---|---|---|
| R | −0.40 | 0.42 | −0.59 | −0.40 | 0.57 | −0.60 | 0.29 | −0.63 | −0.62 | −0.11 | −0.40 | −0.15 | −0.72 |
| | * | ** | *** | * | *** | *** | | *** | *** | | * | | *** |

*: $p<0.05$, **: $p<0.01$, ***: $p<0.001$

EP 4 160 525 A1

# FIG.9

(a1)

VISUAL SCORE ($S_{ss}$) vs SAGGING SKIN COEFFICIENT, R=0.72

(b1)

VISUAL SCORE ($S_{ss}$) vs INTRAFACIAL MOVEMENT INDUCED SAG (cc), R=0.73

(c1)

VISUAL SCORE ($S_{ss}$) vs ANTEROPOSTERIOR BUCCAL SAG (cc), R=0.30, R=0.06

EP 4 160 525 A1

# FIG.10

(a2)

(b2)

(c2)

R=0.66

R=0.64

R=0.10

R=0.09

# FIG.11

## 1 EVALUATION SYSTEM

```
                                          ┌──────────────┐ ⟋10
                                          │  EVALUATION  │
                                          │    DEVICE    │
                                          └──────┬───────┘
         ⟋20                                     │      ⟋30
┌──────────────────┐              ┌──────────────┴───┐
│ IMAGE CAPTURING  │──────────────│    ANALYSIS      │
│    TERMINAL      │              │    TERMINAL      │
└──────────────────┘              └──────────────────┘
```

# FIG.12

10

EVALUATION DEVICE

ACQUISITION UNIT — 101

CALCULATION UNIT — 102

EVALUATION UNIT — 103

OUTPUT UNIT — 104

# FIG.13

20

IMAGE CAPTURING TERMINAL

201

GENERATION UNIT

# FIG.14

30

ANALYSIS TERMINAL

301

CALCULATION UNIT

FIG.15

**20**
IMAGE CAPTURING TERMINAL

**30**
ANALYSIS TERMINAL

**10**
EVALUATION DEVICE

S1
GENERATION OF THREE-DIMENSIONAL FACE IMAGE

TRANSMIT THREE-DIMENSIONAL FACE IMAGES
S2

S3
CALCULATE GRAVITY-INDUCED PROMINENCE AMOUNT AND GRAVITY-INDUCED HOLLOWNESS AMOUNT

TRANSMIT GRAVITY-INDUCED PROMINENCE AMOUNT AND GRAVITY-INDUCED HOLLOWNESS AMOUNT
S4

S5
CALCULATE INTRAFACIAL MOVEMENT INDUCED SAG AMOUNT AND ANTEROPOSTERIOR BUCCAL SAG AMOUNT

OUTPUT RESULT
S6

EP 4 160 525 A1

# FIG.16

```
        ┌─────────────────────────┐
        │   START OF EVALUATION   │
        │       PROCESSING        │
        └─────────────────────────┘
                     │
                     ▼
┌────────────────────────────────────────────────┐
│  ACQUIRE GRAVITY-INDUCED PROMINENCE AMOUNT      │ ～S11
│  AND GRAVITY-INDUCED HOLLOWNESS AMOUNT          │
└────────────────────────────────────────────────┘
                     │
                     ▼
┌────────────────────────────────────────────────┐
│ CALCULATE INTRAFACIAL MOVEMENT INDUCED SAG AMOUNT │ ～S12
│  AND ANTEROPOSTERIOR BUCCAL SAG AMOUNT          │
└────────────────────────────────────────────────┘
                     │
                     ▼
┌────────────────────────────────────────────────┐
│              OUTPUT RESULT                      │ ～S13
└────────────────────────────────────────────────┘
                     │
                     ▼
        ┌─────────────────────────┐
        │   END OF EVALUATION     │
        │       PROCESSING        │
        └─────────────────────────┘
```

# FIG.17

```
┌─────────────────────────────────────────┐
│   START OF EVALUATION PROCESSING FOR     │
│     INTRAFACIAL MOVEMENT INDUCED SAG     │
└─────────────────────────────────────────┘
                    │
                    ▼
┌─────────────────────────────────────────┐
│  ACQUIRE GRAVITY-INDUCED PROMINENCE AMOUNT  │ ～S101
│   AND GRAVITY-INDUCED HOLLOWNESS AMOUNT     │
└─────────────────────────────────────────┘
                    │
                    ▼
┌─────────────────────────────────────────┐
│ CALCULATE INTRAFACIAL MOVEMENT INDUCED SAG AMOUNT │ ～S102
└─────────────────────────────────────────┘
                    │
                    ▼
┌─────────────────────────────────────────┐
│              OUTPUT RESULT               │ ～S103
└─────────────────────────────────────────┘
                    │
                    ▼
┌─────────────────────────────────────────┐
│    END OF EVALUATION PROCESSING FOR      │
│     INTRAFACIAL MOVEMENT INDUCED SAG     │
└─────────────────────────────────────────┘
```

# FIG.18

START OF EVALUATION PROCESSING
FOR INTRAFACIAL MOVEMENT INDUCED SAG

ACQUIRE VISUAL SCORE, ANTEROPOSTERIOR BUCCAL
SAG AMOUNT, AND SAGGING SKIN COEFFICIENT — S111

CALCULATE INTRAFACIAL MOVEMENT INDUCED SAG AMOUNT — S112

OUTPUT RESULT — S113

END OF EVALUATION PROCESSING FOR
INTRAFACIAL MOVEMENT INDUCED SAG

# FIG.19

```
┌─────────────────────────────────────────┐
│     START OF EVALUATION PROCESSING       │
│     FOR ANTEROPOSTERIOR BUCCAL SAG       │
└─────────────────────────────────────────┘
                    │
                    ▼
┌─────────────────────────────────────────┐
│  ACQUIRE GRAVITY-INDUCED PROMINENCE      │    S201
│  AMOUNT AND GRAVITY-INDUCED HOLLOWNESS   │
│  AMOUNT                                  │
└─────────────────────────────────────────┘
                    │
                    ▼
┌─────────────────────────────────────────┐
│  CALCULATE ANTEROPOSTERIOR BUCCAL SAG    │    S202
│  AMOUNT                                  │
└─────────────────────────────────────────┘
                    │
                    ▼
┌─────────────────────────────────────────┐
│              OUTPUT RESULT               │    S203
└─────────────────────────────────────────┘
                    │
                    ▼
┌─────────────────────────────────────────┐
│     END OF EVALUATION PROCESSING         │
│     FOR ANTEROPOSTERIOR BUCCAL SAG       │
└─────────────────────────────────────────┘
```

# FIG.20

```
        ┌─────────────────────────────────────┐
        │  START OF EVALUATION PROCESSING      │
        │  FOR ANTEROPOSTERIOR BUCCAL SAG      │
        └─────────────────────────────────────┘
                          │
                          ▼
┌──────────────────────────────────────────────────────┐
│  ACQUIRE VISUAL SCORE, INTRAFACIAL MOVEMENT INDUCED    │── S211
│  SAG AMOUNT, AND SAGGING SKIN COEFFICIENT              │
└──────────────────────────────────────────────────────┘
                          │
                          ▼
┌──────────────────────────────────────────────────────┐
│  CALCULATE ANTEROPOSTERIOR BUCCAL SAG AMOUNT           │── S212
└──────────────────────────────────────────────────────┘
                          │
                          ▼
┌──────────────────────────────────────────────────────┐
│  OUTPUT RESULT                                         │── S213
└──────────────────────────────────────────────────────┘
                          │
                          ▼
        ┌─────────────────────────────────────┐
        │  END OF EVALUATION PROCESSING        │
        │  FOR ANTEROPOSTERIOR BUCCAL SAG      │
        └─────────────────────────────────────┘
```

# FIG.21

START OF EVALUATION PROCESSING
FOR SAGGING SKIN COEFFICIENT

ACQUIRE INTRAFACIAL MOVEMENT INDUCED SAG AMOUNT — S301

ACQUIRE ANTEROPOSTERIOR BUCCAL SAG AMOUNT — S302

ACQUIRE VISUAL SCORE — S303

CALCULATE SAGGING SKIN COEFFICIENT — S304

OUTPUT RESULT — S305

END OF EVALUATION PROCESSING
FOR SAGGING SKIN COEFFICIENT

# FIG.22

```
┌─────────────────────────────────┐
│      START OF EVALUATION         │
│  PROCESSING FOR VISUAL SCORE     │
└─────────────────────────────────┘
                 │
                 ▼
┌─────────────────────────────────────────────┐
│ ACQUIRE INTRAFACIAL MOVEMENT INDUCED SAG AMOUNT │──S401
└─────────────────────────────────────────────┘
                 │
                 ▼
┌─────────────────────────────────────────────┐
│   ACQUIRE ANTEROPOSTERIOR BUCCAL SAG AMOUNT   │──S402
└─────────────────────────────────────────────┘
                 │
                 ▼
┌─────────────────────────────────────────────┐
│      ACQUIRE SAGGING SKIN COEFFICIENT         │──S403
└─────────────────────────────────────────────┘
                 │
                 ▼
┌─────────────────────────────────────────────┐
│          CALCULATE VISUAL SCORE               │──S404
└─────────────────────────────────────────────┘
                 │
                 ▼
┌─────────────────────────────────────────────┐
│              OUTPUT RESULT                    │──S405
└─────────────────────────────────────────────┘
                 │
                 ▼
┌─────────────────────────────────┐
│      END OF EVALUATION           │
│  PROCESSING FOR VISUAL SCORE     │
└─────────────────────────────────┘
```

FIG.23

EP 4 160 525 A1

# FIG.24

(a)

| i | R0 | R1 | R2 | R3 | R4 | R5 | R6 | R7 | R8 | R9 | Ue | Uv | Ur |
|---|----|----|----|----|----|----|----|----|----|----|----|----|----|
| $a_i$ | −0.995 | 1.725 | −1.169 | −0.977 | 1.634 | −0.924 | 0.854 | −1.225 | −1.478 | −2.468 | −1.122 | −1.945 | −2.452 |
| $b_i$ | 1.053 | 0.412 | 1.489 | 1.078 | 0.353 | 0.998 | 0.321 | 1.000 | 1.117 | 0.680 | 0.987 | 0.809 | 0.970 |

(b)

EP 4 160 525 A1

# FIG.25

(a)

| $i_1,i_2,(i_3)$ | R1,Ur | R5,R8 | R4,R8 | R4,Ur | R4,R5,R8 | R3,R4,R9 |
|---|---|---|---|---|---|---|
| $a_{i1}$ | 0.238 | −0.607 | 1.199 | 0.595 | 1.122 | −1.308 |
| $a_{i2}$ | −2.578 | −1.031 | −1.167 | −2.011 | −0.053 | 2.218 |
| $a_{i3}$ | − | − | − | − | −1.148 | −5.017 |
| b | 1.014 | 1.224 | 0.830 | 0.814 | 0.857 | 1.084 |

(b)

VISUAL SCORE ACQUIRED FROM VISUAL EVALUATION

R=0.821  i=R1,Ur
R=0.827  i=R5,R8
R=0.828  i=R4,R8
R=0.836  i=R4,Ur
R=0.829  i=R4,R5,R8
R=0.833  i=R3,R4,R9

VISUAL SCORE CALCULATED
BY EQUATION (4)

# FIG.26

(1)                              (2)                              (3)

# FIG.27

# FIG.28

3 + 3 IMAGES

3 × 3 ANALYSES

ONE $S_f$ INDEX

# FIG.29

# FIG.30

# FIG.31

EP 4 160 525 A1

## INTERNATIONAL SEARCH REPORT

| | International application No. |
|---|---|
| | PCT/JP2021/019525 |

**A. CLASSIFICATION OF SUBJECT MATTER**
G06T 7/00(2017.01)i; A61B 5/00(2006.01)i; A61B 5/107(2006.01)i
FI: A61B5/107 110; A61B5/107 800; G06T7/00 660A; G06T7/00 C; A61B5/00 M
According to International Patent Classification (IPC) or to both national classification and IPC

**B. FIELDS SEARCHED**

Minimum documentation searched (classification system followed by classification symbols)
G06T7/00; A61B5/00; A61B5/107

Documentation searched other than minimum documentation to the extent that such documents are included in the fields searched
Published examined utility model applications of Japan       1922–1996
Published unexamined utility model applications of Japan     1971–2021
Registered utility model specifications of Japan             1996–2021
Published registered utility model applications of Japan     1994–2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2014-4105 A (ROHTO PHARMACEUTICAL CO., LTD.) 16 January 2014 (2014-01-16) paragraphs [0020]-[0029] | 1-7, 9-18 |
| X | paragraphs [0020]-[0029] | 8 |
| Y | JP 2011-15862 A (POLA CHEMICAL INDUSTRIES, INC.) 27 January 2011 (2011-01-27) paragraphs [0013]-[0017] | 18 |
| Y | JP 2010-51717 A (KAO CORP.) 11 March 2010 (2010-03-11) paragraphs [0009], [0022]-[0024] | 18 |
| A | JP 2019-198597 A (MISAGODO KANPO CO., LTD.) 21 November 2019 (2019-11-21) entire text, all drawings | 1-18 |
| A | JP 10-43141 A (KANEBO KABUSHIKI KAISHA) 17 February 1998 (1998-02-17) entire text, all drawings | 1-18 |
| A | JP 2013-59529 A (SHISEIDO CO., LTD.) 04 April 2013 (2013-04-04) entire text, all drawings | 1-18 |
| A | US 2013/0172691 A1 (BAO, Tran) 04 July 2013 (2013-07-04) paragraph [0132] | 1-18 |

☐ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 June 2021 (29.06.2021) | 13 July 2021 (13.07.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

48

| International application no. |
|---|
| PCT/JP2021/019525 |

| Patent Documents referred in the Report | Publication Date | Patent Family | Publication Date |
|---|---|---|---|
| JP 2014-4105 A | 16 Jan. 2014 | (Family: none) | |
| JP 2011-15862 A | 27 Jan. 2011 | (Family: none) | |
| JP 2010-51717 A | 11 Mar. 2010 | (Family: none) | |
| JP 2019-198597 A | 21 Nov. 2019 | (Family: none) | |
| JP 10-43141 A | 17 Feb. 1998 | (Family: none) | |
| JP 2013-59529 A | 04 Apr. 2013 | (Family: none) | |
| US 2013/0172691 A1 | 04 Jul. 2013 | US 2009/0227876 A1 paragraph [0107] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6473959 B
- JP 2020092470 A

- JP 2020209513 A

**Non-patent literature cited in the description**

- **T. EZURE ; J. HOSOI ; S. AMANO ; T. TSUCHIYA.** *Skin Research and Technology,* 2009, vol. 15, 299-305